# EUROPEAN PATENT APPLICATION

(11) **EP 2 543 725 A1**
(43) Date of publication of application: **09.01.2013**
(21) Application number: 11173306.9
(22) Date of filing: 08.07.2011
(51) Int. Cl.: C12N 9/88

(54) **Polysaccharide lyases**

(71) Applicant: BIOMAY AG, 1090 Wien (AT)
(72) Inventor: Wallner, Michael, 5071 Wals (AT); Hauser, Michael, 6511 Zams (AT); Gadermaier, Gabriele, 5300 Hallwang (AT); Himly, Martin, 9500 Villach (AT); Ferreira, Fatima, 5020 Salzburg (AT)
(74) Representative: Sonn & Partner Patentanwälte

(57) **Abstract**

The present invention relates to a variant of a mature polysaccharide lyase having a beta helix structure, wherein said variant comprises an N- and/or C-terminal truncation and consists essentially of at least a part of the beta helix structure of the mature polysaccharide lyase.

SEQUENCE LISTING

<110>  Biomay AG

<120>  Polysaccharide Lyases

<130>  R 58114

<160>  49

<170>  PatentIn version 3.5

<210>  1
<211>  253
<212>  PRT
<213>  Artificial Sequence

<220>
<223>  Artificial

<400>  1

Ala Asn Pro Lys Glu Gly Thr Leu Arg Phe Ala Ala Ala Gln Asn Arg
1               5                   10                  15

Pro Leu Trp Ile Ile Phe Lys Asn Asp Met Val Ile Asn Leu Asn Gln
            20                  25                  30

Glu Leu Val Val Asn Ser Asp Lys Thr Ile Asp Gly Arg Gly Val Lys
        35                  40                  45

Val Glu Ile Ile Asn Gly Gly Leu Thr Leu Met Asn Val Lys Asn Ile
    50                  55                  60

Ile Ile His Asn Ile Asn Ile His Asp Val Lys Val Leu Pro Gly Gly
65                  70                  75                  80

Met Ile Lys Ser Asn Asp Gly Pro Pro Ile Leu Arg Gln Ala Ser Asp
                85                  90                  95

Gly Asp Thr Ile Asn Val Ala Gly Ser Ser Gln Ile Trp Ile Asp His
            100                 105                 110

Cys Ser Leu Ser Lys Ser Phe Asp Gly Leu Val Asp Val Thr Leu Gly
        115                 120                 125

Ser Thr His Val Thr Ile Ser Asn Cys Lys Phe Thr Gln Gln Ser Lys
    130                 135                 140

Ala Ile Leu Leu Gly Ala Asp Asp Thr His Val Gln Asp Lys Gly Met
145                 150                 155                 160

Leu Ala Thr Val Ala Phe Asn Met Phe Thr Asp Asn Val Asp Gln Arg
                165                 170                 175

Met Pro Arg Cys Arg Phe Gly Phe Phe Gln Val Val Asn Asn Asn Tyr
            180                 185                 190

Asp Arg Trp Gly Thr Tyr Ala Ile Gly Gly Ser Ser Ala Pro Thr Ile
        195                 200                 205

Leu Cys Gln Gly Asn Arg Phe Leu Ala Pro Asp Asp Gln Ile Lys Lys
    210                 215                 220

Asn Val Leu Ala Arg Thr Gly Thr Gly Ala Ala Glu Ser Met Ala Trp
225                 230                 235                 240

Asn Trp Arg Ser Asp Lys Asp Leu Leu Glu Asn Gly Ala
                245                 250

<210>  2
<211>  254
<212>  PRT
<213>  Artificial Sequence

<220>
<223>  Artificial

<400>  2

Ala Pro Gly Thr Leu Arg Tyr Gly Ala Thr Arg Asp Arg Pro Leu Trp
1               5                   10                  15

Ile Ile Phe Ser Gly Asn Met Asn Ile Lys Leu Lys Met Pro Met Tyr
            20                  25                  30

Ile Ala Gly Tyr Lys Thr Phe Asp Gly Arg Gly Ala Gln Val Tyr Ile
        35                  40                  45

Gly Asn Gly Gly Pro Cys Val Phe Ile Lys Arg Val Ser Asn Val Ile
    50                  55                  60

Ile His Gly Leu His Leu Tyr Gly Cys Ser Thr Ser Val Leu Gly Asn
65                  70                  75                  80

Val Leu Ile Asn Glu Ser Phe Gly Val Glu Pro Val His Pro Gln Asp
                85                  90                  95

Gly Asp Ala Leu Thr Leu Arg Thr Ala Thr Asn Ile Trp Ile Asp His
            100                 105                 110

Asn Ser Phe Ser Asn Ser Ser Asp Gly Leu Val Asp Val Thr Leu Ser
        115                 120                 125

Ser Thr Gly Val Thr Ile Ser Asn Asn Leu Phe Phe Asn His His Lys
    130                 135                 140

Val Met Leu Leu Gly His Asp Asp Ala Tyr Ser Asp Asp Lys Ser Met
145                 150                 155                 160

Lys Val Thr Val Ala Phe Asn Gln Phe Gly Pro Asn Cys Gly Gln Arg
                165                 170                 175

Met Pro Arg Ala Arg Tyr Gly Leu Val His Val Ala Asn Asn Asn Tyr
            180                 185                 190

Asp Pro Trp Thr Ile Tyr Ala Ile Gly Gly Ser Ser Asn Pro Thr Ile
        195                 200                 205

Leu Ser Glu Gly Asn Ser Phe Thr Ala Pro Asn Glu Ser Tyr Lys Lys
    210                 215                 220

Gln Val Thr Ile Arg Ile Gly Cys Lys Thr Ser Ser Ser Cys Ser Asn
225                 230                 235                 240

Trp Val Trp Gln Ser Thr Gln Asp Val Phe Tyr Asn Gly Ala
                245                 250

<210>  3
<211>  254
<212>  PRT
<213>  Artificial Sequence

<220>
<223>  Artificial

<400>  3

Thr Pro Gly Thr Leu Arg Tyr Gly Ala Thr Arg Glu Lys Ala Leu Trp
1               5                   10                  15

Ile Ile Phe Ser Gln Asn Met Asn Ile Lys Leu Gln Met Pro Leu Tyr
            20                  25                  30

Val Ala Gly Tyr Lys Thr Ile Asp Gly Arg Gly Ala Val Val His Leu
        35                  40                  45

Gly Asn Gly Gly Pro Cys Leu Phe Met Arg Lys Ala Ser His Val Ile
    50                  55                  60

Leu His Gly Leu His Ile His Gly Cys Asn Thr Ser Val Leu Gly Asp
65                  70                  75                  80

Val Leu Val Ser Glu Ser Ile Gly Val Glu Pro Val His Ala Gln Asp
                85                  90                  95

Gly Asp Ala Ile Thr Met Arg Asn Val Thr Asn Ala Trp Ile Asp His
            100                 105                 110

Asn Ser Leu Ser Asp Cys Ser Asp Gly Leu Ile Asp Val Thr Leu Gly
        115                 120                 125

Ser Thr Gly Ile Thr Ile Ser Asn Asn His Phe Phe Asn His His Lys
    130                 135                 140

Val Met Leu Leu Gly His Asp Asp Thr Tyr Asp Asp Asp Lys Ser Met
145                 150                 155                 160

Lys Val Thr Val Ala Phe Asn Gln Phe Gly Pro Asn Ala Gly Gln Arg
                165                 170                 175

Met Pro Arg Ala Arg Tyr Gly Leu Val His Val Ala Asn Asn Asn Tyr
            180                 185                 190

Asp Gln Trp Asn Ile Tyr Ala Ile Gly Gly Ser Ser Asn Pro Thr Ile
        195                 200                 205

Leu Ser Glu Gly Asn Ser Phe Thr Ala Pro Asn Glu Ser Tyr Lys Lys
    210                 215                 220

Glu Val Thr Lys Arg Ile Gly Cys Glu Thr Thr Ser Ala Cys Ala Asn
225                 230                 235                 240

Trp Val Trp Arg Ser Thr Arg Asp Ala Phe Thr Asn Gly Ala
                245                 250


<210>  4
<211>  254
<212>  PRT
<213>  Artificial Sequence

<220>
<223>  Artificial

<400>  4

Thr Pro Gly Thr Leu Arg Tyr Gly Ala Thr Arg Glu Lys Ala Leu Trp
1               5                   10                  15

Ile Ile Phe Ser Gln Asn Met Asn Ile Lys Leu Lys Met Pro Leu Tyr
            20                  25                  30

Val Ala Gly His Lys Thr Ile Asp Gly Arg Gly Ala Asp Val His Leu
        35                  40                  45

Gly Asn Gly Gly Pro Cys Leu Phe Met Arg Lys Val Ser His Val Ile
    50                  55                  60

Leu His Ser Leu His Ile His Gly Cys Asn Thr Ser Val Leu Gly Asp
65                  70                  75                  80

Val Leu Val Ser Glu Ser Ile Gly Val Glu Pro Val His Ala Gln Asp
                85                  90                  95

Gly Asp Ala Ile Thr Met Arg Asn Val Thr Asn Ala Trp Ile Asp His
            100                 105                 110

Asn Ser Leu Ser Asp Cys Ser Asp Gly Leu Ile Asp Val Thr Leu Gly
        115                 120                 125

Ser Thr Gly Ile Thr Ile Ser Asn Asn His Phe Phe Asn His His Lys
    130                 135                 140

Val Met Leu Leu Gly His Asp Asp Thr Tyr Asp Asp Asp Lys Ser Met
145                 150                 155                 160

Lys Val Thr Val Ala Phe Asn Gln Phe Gly Pro Asn Ala Gly Gln Arg
                165                 170                 175

Met Pro Arg Ala Arg Tyr Gly Leu Val His Val Ala Asn Asn Asn Tyr
            180                 185                 190

Asp Pro Trp Asn Ile Tyr Ala Ile Gly Gly Ser Ser Asn Pro Thr Ile
        195                 200                 205

Leu Ser Glu Gly Asn Ser Phe Thr Ala Pro Ser Glu Ser Tyr Lys Lys
    210                 215                 220

Glu Val Thr Lys Arg Ile Gly Cys Glu Ser Pro Ser Ala Cys Ala Asn
225                 230                 235                 240

Trp Val Trp Arg Ser Thr Arg Asp Ala Phe Ile Asn Gly Ala
                245                 250


<210>  5
<211>  254
<212>  PRT
<213>  Artificial Sequence

<220>
<223>  Artificial

<400>  5

Ala Pro Gly Thr Leu Arg Tyr Gly Ala Thr Arg Glu Arg Ser Leu Trp
1               5                   10                  15

Ile Ile Phe Ser Lys Asn Leu Asn Ile Lys Leu Asn Met Pro Leu Tyr
            20                  25                  30

Ile Ala Gly Asn Lys Thr Ile Asp Gly Arg Gly Ala Glu Val His Ile
        35                  40                  45

Gly Asn Gly Gly Pro Cys Leu Phe Met Arg Thr Val Ser His Val Ile
    50                  55                  60

Leu His Gly Leu Asn Ile His Gly Cys Asn Thr Ser Val Ser Gly Asn
65                  70                  75                  80

Val Leu Ile Ser Glu Ala Ser Gly Val Val Pro Val His Ala Gln Asp
                85                  90                  95

Gly Asp Ala Ile Thr Met Arg Asn Val Thr Asp Val Trp Ile Asp His
            100                 105                 110

Asn Ser Leu Ser Asp Ser Ser Asp Gly Leu Val Asp Val Thr Leu Ala
        115                 120                 125

Ser Thr Gly Val Thr Ile Ser Asn Asn His Phe Phe Asn His His Lys
    130                 135                 140

Val Met Leu Leu Gly His Ser Asp Ile Tyr Ser Asp Asp Lys Ser Met
145                 150                 155                 160

Lys Val Thr Val Ala Phe Asn Gln Phe Gly Pro Asn Ala Gly Gln Arg
                165                 170                 175

Met Pro Arg Ala Arg Tyr Gly Leu Ile His Val Ala Asn Asn Asn Tyr
            180                 185                 190

Asp Pro Trp Ser Ile Tyr Ala Ile Gly Gly Ser Ser Asn Pro Thr Ile
        195                 200                 205

Leu Ser Glu Gly Asn Ser Phe Thr Ala Pro Asn Asp Ser Asp Lys Lys
    210                 215                 220

Glu Val Thr Arg Arg Val Gly Cys Glu Ser Pro Ser Thr Cys Ala Asn
225                 230                 235                 240

Trp Val Trp Arg Ser Thr Gln Asp Ser Phe Asn Asn Gly Ala
                245                 250

<210>  6
<211>  263
<212>  PRT
<213>  Artificial Sequence

<220>
<223>  Artificial

<400>  6

Ala Asn Pro Lys Pro Gly Thr Leu Arg Cys Gly Val Thr Gln Asp Lys
1               5                   10                  15

Pro Leu Trp Ile Ile Phe Lys Lys Asp Met Val Ile Lys Leu Lys His
            20                  25                  30

Glu Leu Val Ile Asn Lys Asp Lys Thr Ile Asp Gly Arg Gly Ala Asn
        35                  40                  45

Val Glu Ile Thr Cys Gly Gly Leu Thr Ile His Asn Val Cys Asn Val
    50                  55                  60

Ile Ile His Asn Ile His Ile His Asp Ile Lys Val Thr Glu Gly Gly
65                  70                  75                  80

Ile Ile Lys Ala Thr Asp Ala Lys Pro Gly His Arg His Lys Ser Asp
                85                  90                  95

Gly Asp Gly Ile Cys Val Ala Gly Ser Ser Lys Ile Trp Ile Asp His
            100                 105                 110

Cys Thr Leu Ser His Gly Pro Asp Gly Leu Ile Asp Val Thr Leu Gly
        115                 120                 125

Ser Thr Ala Val Thr Ile Ser Asn Cys Lys Phe Ser His His Gln Lys
    130                 135                 140

Ile Leu Leu Leu Gly Ala Asp Asn Ser His Val Asp Asp Lys Lys Met
145                 150                 155                 160

His Val Thr Val Ala Phe Asn Arg Phe Ala Glu Ala Cys Asp Gln Arg
                165                 170                 175

Met Pro Arg Cys Arg Phe Gly Phe Phe Gln Val Val Asn Asn Asp Tyr
            180                 185                 190

Thr Ser Trp Gly Thr Tyr Ala Ile Gly Gly Ser Ala Asn Pro Thr Ile
        195                 200                 205

Leu Ser Gln Gly Asn Arg Phe His Ala Pro Asn Asp Pro Met Lys Lys
    210                 215                 220

Asn Val Leu Val Arg Ala Asp Ala Pro His Thr Glu Ser Met Lys Trp
225                 230                 235                 240

Asn Trp Arg Ser Glu Lys Asp Leu Leu Glu Asn Gly Ala Ile Phe Val
                245                 250                 255

Ala Ser Gly Cys Asp Pro His
            260

<210>  7
<211>  759
<212>  DNA
<213>  Artificial Sequence

<220>
<223>  Artificial

<400>  7
gcaaatccaa aagaaggcac actccggttt gctgccgccc aaaacaggcc cttgtggatc      60

atttttaaaa atgatatggt gattaatttg aatcaagagc ttgtcgtaaa cagcgacaag     120

accatcgatg gccgaggagt gaaagttgaa atcattaacg gaggtctcac cctcatgaat     180

gtcaagaata taatcattca taacataaat atccatgatg ttaaagtgct tccaggaggc     240

atgattaagt ccaacgatgg tccaccaatt ttaagacaag caagtgatgg tgatactata     300

aatgttgctg gtagttccca aatatggata gaccattgct cactcagcaa gtctttcgat     360

gggctggtcg atgtcaccct cggtagcaca cacgtgacca tttccaactg caaattcacc     420

caacagtcaa aagcaatatt gttgggggca gatgacaccc atgttcaaga taaaggaatg     480

ctagccacgg tcgctttcaa catgttcacc gataacgttg accaaagaat gcctagatgt     540

cgatttgggt ttttccaagt tgttaacaac aactacgaca gatggggaac gtacgccata     600

ggtggtagct cggccccaac tatactctgc caagggaaca gattcttggc ccctgatgat     660

cagatcaaga aaaatgtcct agcgaggact ggtacaggcg ctgctgagtc gatggcgtgg 720

aactggagat ctgataaaga cttgcttgaa aatggtgct 759

<210> 8
<211> 759
<212> DNA
<213> Artificial Sequence

<220>
<223> Artificial

<400> 8
gcaaacccga aagaaggtac gttacggttt gccgccgccc agaaccgtcc cttatggatt 60

atttttaaaa acgatatggt tattaactta aaccaggagc ttgttgtaaa cagcgacaaa 120

accattgatg gtcgaggtgt taaagtggaa attattaacg gtggcttaac cttaatgaac 180

gttaaaaaca tcattattca taacatcaac attcatgatg tgaaagttct tccgggtggt 240

atgattaaat cgaacgatgg cccgccgatt ttacgtcagg caagcgatgg cgatacgatc 300

aacgtggccg gcagctcgca gatctggatc gaccattgct cgttaagcaa atcgtttgat 360

ggtctagtgg atgtgacctt aggcagcacg cacgttacca tttcgaactg caaatttacc 420

cagcaatcga aagcaatctt attaggtgca gatgacaccc atgtgcagga taaaggtatg 480

ctcgccacgg ttgcctttaa catgtttacc gataacgtgg accagcgtat gcctcgttgc 540

cgatttggtt tttttcaggt ggtgaacaac aactacgacc gttggggtac ttacgccatc 600

ggcggcagct cggccccgac gatcttatgc cagggtaacc gttttttagc ccctgatgat 660

caaattaaaa aaaacgttct cgctcgtacg ggcacgggtg ccgccgagtc gatggcttgg 720

aactggcgtt cggataaaga cttacttgaa aacggcgcc 759

<210> 9
<211> 762
<212> DNA
<213> Artificial Sequence

<220>
<223> Artificial

<400> 9
gcaccaggaa ctctgcgcta tggagcaacc cgagataggc ccctgtggat aattttcagt 60

gggaatatga atataaagct caaaatgcct atgtacattg ctgggtataa gacttttgat 120

ggcaggggag cacaagttta tattggcaat ggcggtccct gtgtgtttat caagagagtt 180

agcaatgtta tcatacacgg tttgtatctg tacggctgta gtactagtgt tttggggaat 240

gttttgataa acgagagttt tggggtggag cctgttcatc ctcaggatgg cgatgctctt 300

actctgcgca ctgctacaaa tatttggatt gatcataatt ctttctccaa ttcttctgat 360

ggtctggtcg atgtcactct tacttcgact ggagttacta tttcaaacaa tctttttttc 420

aaccatcata aagtgatgtt gttagggcat gatgatgcat atagtgatga caaatccatg 480

aaggtgacag tggcgttcaa tcaatttgga cctaactgtg gacaaagaat gcccagggca 540

cgatatggac ttgtacatgt tgcaaacaat aattatgacc catggactat atatgcaatt 600

ggtgggagtt caaatccaac cattctaagt gaagggaata gtttcactgc accaaatgag 660

agctacaaga agcaagtaac catacgtatt ggatgcaaaa catcatcatc ttgttcaaat 720

tgggtgtggc aatctacaca agatgttttt tataatggag ct 762

<210> 10
<211> 762
<212> DNA
<213> Artificial Sequence

<220>
<223> Artificial

<400> 10
ccgggcaccc tgcggtatgg cgccacccga gataggcctc tgtggatcat ttttagcggt 60

aacatgaaca tcaaattaaa aatgccgatg tacattgccg gttataaaac ctttgatggt 120

aggggcgccc aggtgtatat tggtaacggt ggtccttgcg tgtttatcaa acgtgtgagt 180

aacgtgatca tccacggtct gtatctgtac ggttgcagca ccagcgtgct gggtaacgtg 240

ctgatcaatg aaagctttgg tgtggaaccg gtgcatccgc aggatggtga tgccctgacc 300

ctgcggaccg ccaccaacat ttggattgat cataactcgt tttcgaactc gtcggatggt 360

ctggttgatg ttaccctgac ctcgaccggc gtgaccattt cgaataacct gttttttaat 420

catcataaag tgatgctgtt gggtcatgat gatgcctata gcgatgacaa atcgatgaaa 480

gtgaccgtgg cttttaacca gtttggcccg aattgcggcc agcgtatgcc tagggcccga 540

tatggcctgg ttcatgtggc caataacaac tatgacccgt ggaccatcta tgccattggt 600

ggtagctcga acccgaccat tctcagcgaa ggtaacagct ttaccgcccc gaacgaaagt 660

tacaaaaaac aggttaccat ccgaattggc tgcaaaacct cgtcgtcgtg ctcgaactgg 720

gtgtggcagt cgacccagga tgtgttttat aacggcgcct at 762

<210> 11
<211> 762
<212> DNA
<213> Artificial Sequence

<220>
<223> Artificial

<400> 11
acaccaggaa ctttgcgcta tggagcaaca agagaaaaag cactttggat aattttctct 60

cagaatatga atataaagct ccagatgcct ttgtatgttg ctggatataa gactattgac 120

ggcaggggag cagttgttca tcttggcaat ggcggtccct gtctgtttat gaggaaagcg 180

agccatgtta ttctccatgg tttgcatata cacggttgta atacgagtgt tttgggggat 240

gttttggtaa gtgagtccat tggtgtggag cctgttcatg ctcaggatgg ggacgccatt 300

actatgcgga atgttacaaa tgcttggatt gatcataatt ctctctccga ttgttctgat 360

ggtcttatcg atgttacact tggttccact ggaattacta tctccaacaa tcacttcttc 420

aaccatcata aagtgatgtt gttaggacat gatgatacat atgatgatga caaatctatg 480

aaagtgacag tggccttcaa tcaatttgga cccaatgctg ggcaaagaat gccaagggca 540

cgatatggac ttgtacatgt tgcaaacaat aattatgatc aatggaatat atatgctatt 600

ggtgggagtt caaatccaac cattctaagt gaagggaata gtttcactgc tccaaatgag 660

agctacaaaa aggaagtaac aaagcgtata gggtgtgaaa caacatcagc ttgtgcgaac 720

tgggtgtgga gatccacacg agatgctttt actaatggag ct 762

<210> 12
<211> 762
<212> DNA
<213> Artificial Sequence

<220>
<223> Artificial

<400> 12
acccctggca ccttaaggta tggcgcaacc cgagaaaaag cattatggat cattttttcg 60

caaaacatga acatcaaact ccaaatgcct ttatatgtgg ccggctataa aaccattgac 120

ggacgaggcg cagtggtgca tttaggaaac ggaggtccct gcctattcat gcgaaaagct 180

agccatgtga ttctccacgg tttacatatc cacggttgca acacaagcgt gttaggtgat 240

gtgttagtaa gcgaatcgat tggtgtggaa cctgtgcatg cccaagatgg tgacgctatt 300

accatgcgaa acgtgaccaa cgcctggatt gatcataact cgctctcgga ttgctcggat 360

ggtttaatcg atgtgacctt aggttcgacc ggcattacca tctcgaataa ccactttttt 420

aatcatcata aagtgatgtt actaggccat gatgatacct atgatgatga caaatcgatg 480

aaagtgaccg tggcttttaa ccaattcggc cccaacgccg gtcaacgaat gcctcgggca 540

cgatatggct tagtacatgt ggcaaataac aactatgatc aatggaacat ctatgccatt 600

ggtggtagct cgaaccctac gattctaagc gaaggtaaca gctttaccgc ccctaacgaa 660

agctacaaaa aagaagtaac caaaaggatc ggttgcgaaa ccacctcggc ctgcgctaat 720

tgggtgtggc gatcgacccg agatgccttc accaacggcg cc 762

<210> 13
<211> 759
<212> DNA
<213> Artificial Sequence

<220>
<223> Artificial

<400> 13
gcaaatccaa agccagggac acttcgttgt ggtgtcaccc aagataaacc tttgtggatc 60

atctttaaga aagatatggt cataaaactt aaacacgagc ttgtgataaa caaagacaag 120

acaattgatg gaagaggtgc aaatgttgag atcacttgtg gcggtctcac cattcacaac 180

gtttgcaatg tgatcattca taacattcac atacatgata ttaaagtaac cgaaggtgga 240

attattaagg caacggacgc taaaccagga catagacata agagcgacgg agatggtatt 300

tgtgttgctg gttcttcaaa gatatggatc gatcattgca cacttagtca tggtccagat 360

ggccttattg atgtcacgtt gggtagcaca gccgttacca tttccaattg caaatttagc 420

catcaccaaa aaattctatt actcggagca gacaattcac atgtagacga taaaaaaatg 480

catgtcacag tagcattcaa caggttcgca gaagcatgtg atcaaagaat gccacgatgt 540

cgatttggat ttttccaagt tgttaacaat gactacacca gctggggaac gtacgccatt 600

ggtggtagtg ccaatcctac tatccttagc caaggcaacc gattccatgc tccgaatgac 660

ccaatgaaga aaaatgtgtt ggtgagggct gatgcaccac atacagagtc aatgaagtgg 720

aattggagat ctgagaaaga cttgttagaa aatggagct 759

<210> 14
<211> 759
<212> DNA
<213> Artificial Sequence

<220>
<223> Artificial

<400> 14
gccaatccta aacctggaac gttacggtgc ggcgtcaccc aagataaacc gttatggatc 60

atctttaaaa aagatatggt catcaaatta aaacacgagt tagtaatcaa taaagacaaa 120

acgattgatg gccgaggcgc caatgtggag atcacgtgcg gtggccttac cattcacaat 180

gtgtgcaatg taatcattca taatattcac atccatgata ttaaagtaac cgaaggcggc 240

attattaaag ccactgacgc gaaacctggc catcgacata aaagtgacgg cgatggcatt 300

tgcgtggcgg gctcgtcgaa aatctggatc gatcattgca cgttaagtca tggccctgat 360

ggtttaattg atgtcacttt aggcagtacg gcagtgacca tttccaattg caaatttagt 420

catcaccaaa aaattctact acttggcgcc gacaattcgc atgtagacga taaaaaaatg 480

catgtcacgg tagccttcaa tcgattcgcc gaagcctgcg atcaacgaat gcctcgatgc 540

cgatttggct ttttccaagt ggtgaataat gactacacca gttggggcac ttacgcaatt 600

ggcggcagtg caaatccgac gatcttaagt caaggtaatc gattccatgc gcccaatgac 660

cctatgaaaa aaaatgtatt agtacgagcg gatgcccctc atacggagtc gatgaaatgg 720

aattggcgat cggagaaaga cttactcgaa aatggcgcg 759

<210> 15
<211> 762
<212> DNA
<213> Artificial Sequence

<220>
<223> Artificial

<400> 15
acaccaggaa ctttgcgcta tggagcaaca agagaaaaag cactttggat cattttctct 60

cagaatatga atataaagct caagatgcct ttgtatgttg ctggacataa gactattgac 120

ggcaggggag cagatgttca tcttggcaac ggcggtccct gtctgtttat gaggaaagtg 180

agccatgtta ttctccatag tttgcatata cacggttgta atacgagtgt tttgggggat 240

gttttggtaa gtgagtctat tggggtggag cctgttcatg ctcaggatgg ggacgccatt 300

actatgcgca atgttacaaa tgcttggatt gatcataatt ctctctccga ttgttctgat 360

ggtcttatcg atgttacgct tggctccact ggaattacta tctccaacaa tcacttcttc 420

aaccatcata aagtgatgtt gttaggacat gatgatacat atgacgatga caaatctatg 480

aaagtgacag tggcgttcaa tcaatttgga cctaatgctg ggcaaagaat gccaagggca 540

cgatatggac ttgtacatgt cgcaaacaat aattatgatc catggaatat atatgctatt 600

ggtgggagtt caaatccaac cattctaagt gaagggaata gtttcactgc cccaagtgag 660

agctacaaga aggaagtaac aaagcgtata gggtgtgaat caccatcagc ttgtgcgaac 720

tgggtgtgga gatctacacg agatgctttt attaatggag ct 762

<210> 16
<211> 762
<212> DNA
<213> Artificial Sequence

<220>
<223> Artificial

<400> 16
accccgggca ccttaaggta cggcgccacc cgagagaaag ccttatggat catttttagc 60

caaaacatga acatcaaact caaaatgccg ttatacgtgg ccggccataa aaccattgac 120

ggacgaggcg ccgatgtgca tttaggaaat ggaggaccct gcctatttat gcgaaaagtt 180

agccatgtga ttctccatag cttacatatc cacggatgca acacgagcgt gttaggtgat 240

gtgttagtta gcgagagcat tggtgttgag ccggtgcatg cccaagatgg tgacgctatt 300

accatgagga acgtgaccaa cgcctggatt gatcataaca gcctctcgga ttgcagcgat 360

ggattaatcg atgtgacgtt aggatcgacc ggcattacca tctcgaataa ccactttttt 420

aatcatcata aagttatgtt actcggccat gatgatacct acgacgatga caaaagcatg 480

aaagttaccg ttgcttttaa ccaatttggc ccgaacgccg gtcaacgaat gccgcgagcc 540

cgatacggct tagttcatgt agccaataac aactacgatc cgtggaacat ctacgccatt 600

ggaggtagct cgaacccgac gattctaagc gagggtaaca gctttaccgc tccgagcgag 660

agctacaaaa aagaggttac caaacggatc ggttgcgagt cgccgtcggc ctgcgctaat 720

tgggtttggc gatcgacccg agatgccttt attaacggcg cc 762

<210> 17
<211> 762
<212> DNA
<213> Artificial Sequence

<220>
<223> Artificial

<400> 17
gcaccaggta ctctgcgcta cggggcaaca cgagaaaggt cactgtggat cattttctct 60

aagaatctga acataaagct caacatgcct ttgtacattg ctgggaataa gaccattgat 120

ggcagaggag cagaagttca tattggcaat ggtggtccct gtctgtttat gaggacagtg 180

agccatgtta ttctacacgg attgaatata cacggctgta atacaagtgt ttcggggaat 240

gttttgataa gcgaggcttc tggagtggtg cctgttcatg ctcaggatgg cgacgccatt 300

actatgcgca atgttacaga tgtttggatt gatcataatt ctctctccga ttcttctgat 360

ggtcttgtcg atgttacact tgcttccacc ggagttacta tttccaacaa tcattttttc 420

aaccatcata aagtgatgtt gttagggcat agtgatatat atagtgatga caaaagtatg 480

aaggtgacag tggcattcaa tcaatttgga cctaatgctg gacaacgaat gccaagggca 540

cgatatggac ttatacatgt tgcaaacaat aattatgacc catggagtat atatgctatt 600

ggtgggagtt caaatccaac cattctaagt gaagggaata gtttcactgc accaaatgat 660

agcgacaaga aggaagtaac aagacgtgta gggtgtgaat caccatcaac ttgtgcaaac 720

tgggtgtgga gatctacaca agattctttt aataatggag ct 762

<210> 18
<211> 762
<212> DNA
<213> Artificial Sequence

<220>
<223> Artificial

<400> 18
gcccctggaa ccctccgata cggagccacc cgagaacgat cgctctggat aattttcagc 60

aagaacctca atatcaagct taatatgcct ttatacattg ccggaaacaa gacgattgat 120

ggacgtggtg ccgaagtgca tattggaaac ggaggaccct gcctctttat gcgaaccgtc 180

tcgcatgtga ttctccacgg tttaaacatc cacggatgca acaccagcgt gtccggaaac 240

gtgttaatct cggaggccag cggtgtcgtc cctgtgcatg cccaagatgg agacgctatt 300

accatgcgaa acgtgaccga tgtgtggatt gatcataaca gcctttcgga tagcagcgat 360

ggattagtag atgtgacctt agcctcgacg ggtgtgacca tttcgaataa ccattttttc 420

aatcatcata aagtcatgtt attaggacat agcgatatct atagcgatga caaaagcatg 480

aaggtcaccg tcgccttcaa ccagtttggt cctaacgccg gtcagcgaat gcctcgagcc 540

cgatatggtt taatccatgt ggccaataac aactatgacc cttggagcat ctatgccatt 600

ggaggaagca gcaaccctac tattctcagc gaaggaaaca gcttcaccgc ccctaacgat 660

tcggacaaga aggaagtaac ccgtcgagta ggatgcgaaa gccctagcac ctgcgccaat 720

tgggtctggc gtagcaccca ggatagcttt aacaacggtg cc 762

<210> 19
<211> 372
<212> PRT
<213> Artificial Sequence

<220>
<223> Artificial

<400> 19

Ala Glu Gly Val Gly Glu Ile Leu Pro Ser Val Asn Glu Thr Arg Ser
1               5                   10                  15

Leu Gln Ala Cys Glu Ala Leu Asn Ile Ile Asp Lys Cys Trp Arg Gly
            20                  25                  30

Lys Ala Asp Trp Glu Asn Asn Arg Gln Ala Leu Ala Asp Cys Ala Gln
        35                  40                  45

Gly Phe Ala Lys Gly Thr Tyr Gly Gly Lys Trp Gly Asp Val Tyr Thr
    50                  55                  60

Val Thr Ser Asn Leu Asp Asp Asp Val Ala Asn Pro Lys Glu Gly Thr
65                  70                  75                  80

Leu Arg Phe Ala Ala Ala Gln Asn Arg Pro Leu Trp Ile Ile Phe Lys
                85                  90                  95

Asn Asp Met Val Ile Asn Leu Asn Gln Glu Leu Val Val Asn Ser Asp
            100                 105                 110

Lys Thr Ile Asp Gly Arg Gly Val Lys Val Glu Ile Ile Asn Gly Gly
        115                 120                 125

Leu Thr Leu Met Asn Val Lys Asn Ile Ile Ile His Asn Ile Asn Ile
    130                 135                 140

His Asp Val Lys Val Leu Pro Gly Gly Met Ile Lys Ser Asn Asp Gly
145                 150                 155                 160

Pro Pro Ile Leu Arg Gln Ala Ser Asp Gly Asp Thr Ile Asn Val Ala
                165                 170                 175

Gly Ser Ser Gln Ile Trp Ile Asp His Cys Ser Leu Ser Lys Ser Phe
            180                 185                 190

Asp Gly Leu Val Asp Val Thr Leu Gly Ser Thr His Val Thr Ile Ser
        195                 200                 205

Asn Cys Lys Phe Thr Gln Gln Ser Lys Ala Ile Leu Leu Gly Ala Asp
    210                 215                 220

Asp Thr His Val Gln Asp Lys Gly Met Leu Ala Thr Val Ala Phe Asn
225                 230                 235                 240

Met Phe Thr Asp Asn Val Asp Gln Arg Met Pro Arg Cys Arg Phe Gly
                245                 250                 255

Phe Phe Gln Val Val Asn Asn Asn Tyr Asp Arg Trp Gly Thr Tyr Ala
            260                 265                 270

Ile Gly Gly Ser Ser Ala Pro Thr Ile Leu Cys Gln Gly Asn Arg Phe
        275                 280                 285

Leu Ala Pro Asp Asp Gln Ile Lys Lys Asn Val Leu Ala Arg Thr Gly
    290                 295                 300

Thr Gly Ala Ala Glu Ser Met Ala Trp Asn Trp Arg Ser Asp Lys Asp
305                 310                 315                 320

Leu Leu Glu Asn Gly Ala Ile Phe Val Thr Ser Gly Ser Asp Pro Val
                325                 330                 335

Leu Thr Pro Val Gln Ser Ala Gly Met Ile Pro Ala Glu Pro Gly Glu
            340                 345                 350

Ala Ala Ile Lys Leu Thr Ser Ser Ala Gly Val Phe Ser Cys His Pro
        355                 360                 365

Gly Ala Pro Cys
    370

<210>  20
<211>  353
<212>  PRT
<213>  Artificial Sequence

<220>
<223>  Artificial

<400>  20

Asp Asn Pro Ile Asp Ser Cys Trp Arg Gly Asp Ser Asn Trp Ala Gln
1               5                   10                  15

Asn Arg Met Lys Leu Ala Asp Cys Ala Val Gly Phe Gly Ser Ser Thr
            20                  25                  30

Met Gly Gly Lys Gly Gly Asp Leu Tyr Thr Val Thr Asn Ser Asp Asp
        35                  40                  45

Asp Pro Val Asn Pro Ala Pro Gly Thr Leu Arg Tyr Gly Ala Thr Arg
    50                  55                  60

Asp Arg Pro Leu Trp Ile Ile Phe Ser Gly Asn Met Asn Ile Lys Leu
65                  70                  75                  80

Lys Met Pro Met Tyr Ile Ala Gly Tyr Lys Thr Phe Asp Gly Arg Gly
                85                  90                  95

Ala Gln Val Tyr Ile Gly Asn Gly Gly Pro Cys Val Phe Ile Lys Arg
            100                 105                 110

Val Ser Asn Val Ile Ile His Gly Leu His Leu Tyr Gly Cys Ser Thr
        115                 120                 125

Ser Val Leu Gly Asn Val Leu Ile Asn Glu Ser Phe Gly Val Glu Pro
    130                 135                 140

Val His Pro Gln Asp Gly Asp Ala Leu Thr Leu Arg Thr Ala Thr Asn
145                 150                 155                 160

Ile Trp Ile Asp His Asn Ser Phe Ser Asn Ser Ser Asp Gly Leu Val
                165                 170                 175

Asp Val Thr Leu Ser Ser Thr Gly Val Thr Ile Ser Asn Asn Leu Phe
            180                 185                 190

Phe Asn His His Lys Val Met Leu Leu Gly His Asp Asp Ala Tyr Ser
        195                 200                 205

Asp Asp Lys Ser Met Lys Val Thr Val Ala Phe Asn Gln Phe Gly Pro
    210                 215                 220

Asn Cys Gly Gln Arg Met Pro Arg Ala Arg Tyr Gly Leu Val His Val
225                 230                 235                 240

Ala Asn Asn Asn Tyr Asp Pro Trp Thr Ile Tyr Ala Ile Gly Gly Ser
                245                 250                 255

Ser Asn Pro Thr Ile Leu Ser Glu Gly Asn Ser Phe Thr Ala Pro Asn
            260                 265                 270

Glu Ser Tyr Lys Lys Gln Val Thr Ile Arg Ile Gly Cys Lys Thr Ser
        275                 280                 285

Ser Ser Cys Ser Asn Trp Val Trp Gln Ser Thr Gln Asp Val Phe Tyr
    290                 295                 300

Asn Gly Ala Tyr Phe Val Ser Ser Gly Lys Tyr Glu Gly Gly Asn Ile
305                 310                 315                 320

Tyr Thr Lys Lys Glu Ala Phe Asn Val Glu Asn Gly Asn Ala Thr Pro
                325                 330                 335

Gln Leu Thr Lys Asn Ala Gly Val Leu Thr Cys Ser Leu Ser Lys Arg
            340                 345                 350

Cys

<210>  21
<211>  346
<212>  PRT
<213>  Artificial Sequence

<220>
<223>  Artificial

<400>  21

Asp Asn Pro Ile Asp Ser Cys Trp Arg Gly Asp Ser Asn Trp Asp Gln
1               5                   10                  15

Asn Arg Met Lys Leu Ala Asp Cys Val Val Gly Phe Gly Ser Ser Thr
            20                  25                  30

Met Gly Gly Lys Gly Gly Glu Ile Tyr Thr Val Thr Ser Ser Glu Asp
        35                  40                  45

Asn Pro Val Asn Pro Thr Pro Gly Thr Leu Arg Tyr Gly Ala Thr Arg
    50                  55                  60

Glu Lys Ala Leu Trp Ile Ile Phe Ser Gln Asn Met Asn Ile Lys Leu
65                  70                  75                  80

Gln Met Pro Leu Tyr Val Ala Gly Tyr Lys Thr Ile Asp Gly Arg Gly
                85                  90                  95

Ala Val Val His Leu Gly Asn Gly Gly Pro Cys Leu Phe Met Arg Lys
            100                 105                 110

Ala Ser His Val Ile Leu His Gly Leu His Ile His Gly Cys Asn Thr
        115                 120                 125

Ser Val Leu Gly Asp Val Leu Val Ser Glu Ser Ile Gly Val Glu Pro
    130                 135                 140

Val His Ala Gln Asp Gly Asp Ala Ile Thr Met Arg Asn Val Thr Asn
145                 150                 155                 160

Ala Trp Ile Asp His Asn Ser Leu Ser Asp Cys Ser Asp Gly Leu Ile
                165                 170                 175

Asp Val Thr Leu Gly Ser Thr Gly Ile Thr Ile Ser Asn Asn His Phe
            180                 185                 190

Phe Asn His His Lys Val Met Leu Leu Gly His Asp Asp Thr Tyr Asp
        195                 200                 205

Asp Asp Lys Ser Met Lys Val Thr Val Ala Phe Asn Gln Phe Gly Pro
    210                 215                 220

Asn Ala Gly Gln Arg Met Pro Arg Ala Arg Tyr Gly Leu Val His Val
225                 230                 235                 240

Ala Asn Asn Asn Tyr Asp Gln Trp Asn Ile Tyr Ala Ile Gly Gly Ser
                245                 250                 255

Ser Asn Pro Thr Ile Leu Ser Glu Gly Asn Ser Phe Thr Ala Pro Asn
            260                 265                 270

Glu Ser Tyr Lys Lys Glu Val Thr Lys Arg Ile Gly Cys Glu Thr Thr
        275                 280                 285

Ser Ala Cys Ala Asn Trp Val Trp Arg Ser Thr Arg Asp Ala Phe Thr
    290                 295                 300

Asn Gly Ala Tyr Phe Val Ser Ser Gly Lys Ala Glu Asp Thr Asn Ile
305                 310                 315                 320

Tyr Asn Ser Asn Glu Ala Phe Lys Val Glu Asn Gly Asn Ala Ala Pro
                325                 330                 335

Gln Leu Thr Gln Asn Ala Gly Val Val Ala
            340                 345

<210>  22
<211>  346
<212>  PRT
<213>  Artificial Sequence

<220>
<223>  Artificial

<400>  22

Asp Asn Pro Ile Asp Ser Cys Trp Arg Gly Asp Ser Asn Trp Asp Gln
1               5                   10                  15

Asn Arg Met Lys Leu Ala Asp Cys Ala Val Gly Phe Gly Ser Ser Thr
            20                  25                  30

Met Gly Gly Lys Gly Gly Asp Phe Tyr Thr Val Thr Ser Thr Asp Asp
        35                  40                  45

Asn Pro Val Asn Pro Thr Pro Gly Thr Leu Arg Tyr Gly Ala Thr Arg
    50                  55                  60

Glu Lys Ala Leu Trp Ile Ile Phe Ser Gln Asn Met Asn Ile Lys Leu
65                  70                  75                  80

Lys Met Pro Leu Tyr Val Ala Gly His Lys Thr Ile Asp Gly Arg Gly
                85                  90                  95

Ala Asp Val His Leu Gly Asn Gly Gly Pro Cys Leu Phe Met Arg Lys
            100                 105                 110

Val Ser His Val Ile Leu His Ser Leu His Ile His Gly Cys Asn Thr
        115                 120                 125

Ser Val Leu Gly Asp Val Leu Val Ser Glu Ser Ile Gly Val Glu Pro
    130                 135                 140

Val His Ala Gln Asp Gly Asp Ala Ile Thr Met Arg Asn Val Thr Asn
145                 150                 155                 160

Ala Trp Ile Asp His Asn Ser Leu Ser Asp Cys Ser Asp Gly Leu Ile
                165                 170                 175

Asp Val Thr Leu Gly Ser Thr Gly Ile Thr Ile Ser Asn Asn His Phe
            180                 185                 190

Phe Asn His His Lys Val Met Leu Leu Gly His Asp Asp Thr Tyr Asp
        195                 200                 205

Asp Asp Lys Ser Met Lys Val Thr Val Ala Phe Asn Gln Phe Gly Pro
    210                 215                 220

Asn Ala Gly Gln Arg Met Pro Arg Ala Arg Tyr Gly Leu Val His Val
225                 230                 235                 240

Ala Asn Asn Asn Tyr Asp Pro Trp Asn Ile Tyr Ala Ile Gly Gly Ser
                245                 250                 255

Ser Asn Pro Thr Ile Leu Ser Glu Gly Asn Ser Phe Thr Ala Pro Ser
            260                 265                 270

Glu Ser Tyr Lys Lys Glu Val Thr Lys Arg Ile Gly Cys Glu Ser Pro
        275                 280                 285

Ser Ala Cys Ala Asn Trp Val Trp Arg Ser Thr Arg Asp Ala Phe Ile
    290                 295                 300

Asn Gly Ala Tyr Phe Val Ser Ser Gly Lys Thr Glu Glu Thr Asn Ile
305                 310                 315                 320

Tyr Asn Ser Asn Glu Ala Phe Lys Val Glu Asn Gly Asn Ala Ala Pro
                325                 330                 335

Gln Leu Thr Lys Asn Ala Gly Val Val Thr
            340                 345


<210>  23
<211>  354
<212>  PRT
<213>  Artificial Sequence

<220>
<223>  Artificial

<400>  23

Asp Asn Pro Ile Asp Ser Cys Trp Arg Gly Asp Ala Asn Trp Asp Gln
1               5                   10                  15

Asn Arg Met Lys Leu Ala Asp Cys Ala Val Gly Phe Gly Ser Ser Ala
            20                  25                  30

Met Gly Gly Lys Gly Gly Ala Phe Tyr Thr Val Thr Ser Ser Asp Asp
        35                  40                  45

Asp Pro Val Asn Pro Ala Pro Gly Thr Leu Arg Tyr Gly Ala Thr Arg
    50                  55                  60

Glu Arg Ser Leu Trp Ile Ile Phe Ser Lys Asn Leu Asn Ile Lys Leu
65                  70                  75                  80

Asn Met Pro Leu Tyr Ile Ala Gly Asn Lys Thr Ile Asp Gly Arg Gly
                85                  90                  95

Ala Glu Val His Ile Gly Asn Gly Gly Pro Cys Leu Phe Met Arg Thr
            100                 105                 110

Val Ser His Val Ile Leu His Gly Leu Asn Ile His Gly Cys Asn Thr
        115                 120                 125

Ser Val Ser Gly Asn Val Leu Ile Ser Glu Ala Ser Gly Val Val Pro
    130                 135                 140

Val His Ala Gln Asp Gly Asp Ala Ile Thr Met Arg Asn Val Thr Asp
145                 150                 155                 160

Val Trp Ile Asp His Asn Ser Leu Ser Asp Ser Ser Asp Gly Leu Val
                165                 170                 175

Asp Val Thr Leu Ala Ser Thr Gly Val Thr Ile Ser Asn Asn His Phe
            180                 185                 190

Phe Asn His His Lys Val Met Leu Leu Gly His Ser Asp Ile Tyr Ser
        195                 200                 205

Asp Asp Lys Ser Met Lys Val Thr Val Ala Phe Asn Gln Phe Gly Pro
    210                 215                 220

Asn Ala Gly Gln Arg Met Pro Arg Ala Arg Tyr Gly Leu Ile His Val
225                 230                 235                 240

Ala Asn Asn Asn Tyr Asp Pro Trp Ser Ile Tyr Ala Ile Gly Gly Ser
                245                 250                 255

Ser Asn Pro Thr Ile Leu Ser Glu Gly Asn Ser Phe Thr Ala Pro Asn
            260                 265                 270

Asp Ser Asp Lys Lys Glu Val Thr Arg Arg Val Gly Cys Glu Ser Pro
        275                 280                 285

Ser Thr Cys Ala Asn Trp Val Trp Arg Ser Thr Gln Asp Ser Phe Asn
    290                 295                 300

Asn Gly Ala Tyr Phe Val Ser Ser Gly Lys Asn Glu Gly Thr Asn Ile
305                 310                 315                 320

Tyr Asn Asn Asn Glu Ala Phe Lys Val Glu Asn Gly Ser Ala Ala Pro
                325                 330                 335

Gln Leu Thr Lys Asn Ala Gly Val Leu Thr Cys Ile Leu Ser Lys Pro
            340                 345                 350

Cys Ser

<210>  24
<211>  376

<212>  PRT
<213>  Artificial Sequence

<220>
<223>  Artificial

<400>  24

Ala Arg Ala Asp Ile Gly Asp Glu Leu Glu Ala Ala Gln Phe Asn Ser
1               5                   10                  15

Thr Arg Arg Gly Leu His Glu Cys Ala Ala His Asn Ile Ile Asp Lys
            20                  25                  30

Cys Trp Arg Cys Lys Ala Asp Trp Glu Lys Asn Arg Gln Ala Leu Ala
        35                  40                  45

Lys Cys Ala Gln Gly Phe Ala Lys Gly Thr Thr Gly Gly Leu Gly Gly
    50                  55                  60

Glu Ile Tyr Val Val Thr Asp Cys Ser Asp Asp Asn Ala Ala Asn Pro
65                  70                  75                  80

Lys Pro Gly Thr Leu Arg Cys Gly Val Thr Gln Asp Lys Pro Leu Trp
                85                  90                  95

Ile Ile Phe Lys Lys Asp Met Val Ile Lys Leu Lys His Glu Leu Val
            100                 105                 110

Ile Asn Lys Asp Lys Thr Ile Asp Gly Arg Gly Ala Asn Val Glu Ile
        115                 120                 125

Thr Cys Gly Gly Leu Thr Ile His Asn Val Cys Asn Val Ile Ile His
    130                 135                 140

Asn Ile His Ile His Asp Ile Lys Val Thr Glu Gly Gly Ile Ile Lys
145                 150                 155                 160

Ala Thr Asp Ala Lys Pro Gly His Arg His Lys Ser Asp Gly Asp Gly
                165                 170                 175

Ile Cys Val Ala Gly Ser Ser Lys Ile Trp Ile Asp His Cys Thr Leu
            180                 185                 190

Ser His Gly Pro Asp Gly Leu Ile Asp Val Thr Leu Gly Ser Thr Ala
        195                 200                 205

Val Thr Ile Ser Asn Cys Lys Phe Ser His His Gln Lys Ile Leu Leu
    210                 215                 220

Leu Gly Ala Asp Asn Ser His Val Asp Asp Lys Lys Met His Val Thr
225                 230                 235                 240

Val Ala Phe Asn Arg Phe Ala Glu Ala Cys Asp Gln Arg Met Pro Arg
                245                 250                 255

Cys Arg Phe Gly Phe Phe Gln Val Val Asn Asn Asp Tyr Thr Ser Trp
            260                 265                 270

Gly Thr Tyr Ala Ile Gly Gly Ser Ala Asn Pro Thr Ile Leu Ser Gln
        275                 280                 285

Gly Asn Arg Phe His Ala Pro Asn Asp Pro Met Lys Lys Asn Val Leu
    290                 295                 300

Val Arg Ala Asp Ala Pro His Thr Glu Ser Met Lys Trp Asn Trp Arg
305                 310                 315                 320

Ser Glu Lys Asp Leu Leu Glu Asn Gly Ala Ile Phe Val Ala Ser Gly
                325                 330                 335

Cys Asp Pro His Leu Thr Pro Glu Gln Lys Ser His Leu Ile Pro Ala
            340                 345                 350

Glu Pro Gly Ser Ala Val Leu Gln Leu Thr Ser Cys Ala Gly Thr Leu
        355                 360                 365

Lys Cys Val Pro Gly Lys Pro Cys
    370                 375

<210>  25
<211>  1119
<212>  DNA
<213>  Artificial Sequence

<220>
<223>  Artificial

<400>  25
gccgaagggg tcggagaaat cttaccttca gttaacgaaa cgaggagcct gcaagcatgt      60

gaagcataca acattataga caagtgctgg aggggcaaag ccgattggga gaacaaccga     120

caagcgttag ccgactgtgc ccaaggtttt gcaaagggaa cctacggcgg aaaatggggt     180

gatgtctaca cggtcaccag caatctagat gatgatgttg caaatccaaa agaaggcaca     240

ctccggtttg ctgccgccca aaacaggccc ttgtggatca tttttaaaaa tgatatggtg     300

attaatttga atcaagagct tgtcgtaaac agcgacaaga ccatcgatgg ccgaggagtg     360

aaagttgaaa tcattaacgg aggtctcacc ctcatgaatg tcaagaatat aatcattcat     420

aacataaata tccatgatgt taaagtgctt ccaggaggca tgattaagtc caacgatggt     480

ccaccaattt taagacaagc aagtgatggt gatactataa atgttgctgg tagttcccaa     540

atatggatag accattgctc actcagcaag tctttcgatg ggctggtcga tgtcaccctc     600

ggtagcacac acgtgaccat ttccaactgc aaattcaccc aacagtcaaa agcaatattg     660

ttgggggcag atgacaccca tgttcaagat aaaggaatgc tagccacggt cgctttcaac     720

atgttcaccg ataacgttga ccaaagaatg cctagatgtc gatttgggtt tttccaagtt     780

gttaacaaca actacgacag atggggaacg tacgccatag gtggtagctc ggccccaact     840

atactctgcc aagggaacag attcttggcc cctgatgatc agatcaagaa aaatgtccta 900

gcgaggactg gtacaggcgc tgctgagtcg atggcgtgga actggagatc tgataaagac 960

ttgcttgaaa atggtgctat ttttgttaca tctgggtctg atccagtgct aacccctgtt 1020

caaagcgcag ggatgattcc agctgaacca ggagaagccg ctataaaact cactagtagt 1080

gctggtgtac tctcatgccg tcctggagca ccttgctaa 1119

<210> 26
<211> 1119
<212> DNA
<213> Artificial Sequence

<220>
<223> Artificial

<400> 26
gccgaaggtg ttggtgaaat tttaccttcg gtgaacgaaa ctcgtagcct acaggcatgc 60

gaagcataca acattatcga caaatgctgg cgtggtaaag ccgattggga gaacaaccga 120

caggctttag ccgactgcgc ccagggcttt gcaaaaggta cctacggtgg taaatggggc 180

gatgtttaca cggttaccag caacctcgat gatgatgtgg caaacccgaa agaaggtacg 240

ttacggtttg ccgccgccca gaaccgtccc ttatggatta tttttaaaaa cgatatggtt 300

attaacttaa accaggagct tgttgtaaac agcgacaaaa ccattgatgg tcgaggtgtt 360

aaagtggaaa ttattaacgg tggcttaacc ttaatgaacg ttaaaaacat cattattcat 420

aacatcaaca ttcatgatgt gaaagttctt ccgggtggta tgattaaatc gaacgatggc 480

ccgccgattt tacgtcaggc aagcgatggc gatacgatca acgtggccgg cagctcgcag 540

atctggatcg accattgctc gttaagcaaa tcgtttgatg gtctagtgga tgtgacctta 600

ggcagcacgc acgttaccat ttcgaactgc aaatttaccc agcaatcgaa agcaatctta 660

ttaggtgcag atgacaccca tgtgcaggat aaaggtatgc tcgccacggt tgcctttaac 720

atgtttaccg ataacgtgga ccagcgtatg cctcgttgcc gatttggttt ttttcaggtg 780

gtgaacaaca actacgaccg ttggggtact tacgccatcg gcggcagctc ggccccgacg 840

atcttatgcc agggtaaccg ttttttagcc cctgatgatc aaattaaaaa aaacgttctc 900

gctcgtacgg gcacgggtgc cgccgagtcg atggcttgga actggcgttc ggataaagac 960

ttacttgaaa acggcgccat ttttgtgacg tcgggttcgg atccggttct cacccctgtg 1020

cagagcgcag gtatgattcc ggccgaaccg ggtgaagccg ccatcaaatt aacgagcagc 1080

gccggcgtat tatcgtgccg acctggtgca ccttgctaa 1119

<210> 27
<211> 1062
<212> DNA
<213> Artificial Sequence

<220>
<223> Artificial

<400> 27
gataatccca tagacagctg ctggagagga gactcaaact gggcacaaaa cagaatgaag 60

ctcgcagatt gtgcagtggg cttcggaagc tccaccatgg gaggcaaggg aggagatctt 120

tatacggtca cgaactcaga tgacgaccct gtgaatcctg caccaggaac tctgcgctat 180

ggagcaaccc gagataggcc cctgtggata attttcagtg ggaatatgaa tataaagctc 240

aaaatgccta tgtacattgc tgggtataag acttttgatg gcaggggagc acaagtttat 300

attggcaatg gcggtccctg tgtgtttatc aagagagtta gcaatgttat catacacggt 360

ttgtatctgt acggctgtag tactagtgtt ttggggaatg ttttgataaa cgagagtttt 420

ggggtggagc ctgttcatcc tcaggatggc gatgctctta ctctgcgcac tgctacaaat 480

atttggattg atcataattc tttctccaat tcttctgatg gtctggtcga tgtcactctt 540

acttcgactg gagttactat ttcaaacaat ctttttttca accatcataa agtgatgttg 600

ttagggcatg atgatgcata tagtgatgac aaatccatga aggtgacagt ggcgttcaat 660

caatttggac ctaactgtgg acaaagaatg cccagggcac gatatggact tgtacatgtt 720

gcaaacaata attatgaccc atggactata tatgcaattg gtgggagttc aaatccaacc 780

attctaagtg aagggaatag tttcactgca ccaaatgaga gctacaagaa gcaagtaacc 840

atacgtattg gatgcaaaac atcatcatct tgttcaaatt gggtgtggca atctacacaa 900

gatgtttttt ataatggagc ttattttgta tcatcaggga aatatgaagg gggtaatata 960

tacacaaaga aagaagcttt caatgttgag aatgggaatg caactcctca attgacaaaa 1020

aatgctgggg ttttaacatg ctctctctct aaacgttgtt ga 1062

<210> 28
<211> 1062
<212> DNA
<213> Artificial Sequence

<220>
<223> Artificial

<400> 28
gataacccta tcgacagttg ctggcgtggc gactcgaatt gggcccagaa tcgtatgaaa 60

ttagccgatt gcgccgtggg ttttggcagt tcgacgatgg gcggtaaagg cggcgatctg 120

tatacagtta caaattcgga tgacgacccg gtgaacccgg ccccgggcac cctgcggtat 180

ggcgccaccc gagataggcc tctgtggatc atttttagcg gtaacatgaa catcaaatta 240

aaaatgccga tgtacattgc cggttataaa acctttgatg gtaggggcgc ccaggtgtat 300

attggtaacg gtggtccttg cgtgtttatc aaacgtgtga gtaacgtgat catccacggt 360

ctgtatctgt acggttgcag caccagcgtg ctgggtaacg tgctgatcaa tgaaagcttt 420

ggtgtggaac cggtgcatcc gcaggatggt gatgccctga ccctgcggac cgccaccaac 480

atttggattg atcataactc gttttcgaac tcgtcggatg gtctggttga tgttaccctg 540

acctcgaccg gcgtgaccat ttcgaataac ctgtttttta atcatcataa agtgatgctg 600

ttgggtcatg atgatgccta tagcgatgac aaatcgatga aagtgaccgt ggcttttaac 660

cagtttggcc cgaattgcgg ccagcgtatg cctagggccc gatatggcct ggttcatgtg 720

gccaataaca actatgaccc gtggaccatc tatgccattg gtggtagctc gaacccgacc 780

attctcagcg aaggtaacag ctttaccgcc ccgaacgaaa gttacaaaaa acaggttacc 840

atccgaattg gctgcaaaac ctcgtcgtcg tgctcgaact gggtgtggca gtcgacccag 900

gatgtgtttt ataacggcgc ctattttgtt tcgtcgggta aatatgaagg tggtaacatc 960

tacaccaaaa aagaagcctt taacgtggaa aacggtaacg ccaccccgca gctgaccaaa 1020

aacgccggtg tgttgacctg ctcgttatcg aaacgatgct ga 1062

<210> 29
<211> 1041
<212> DNA
<213> Artificial Sequence

<220>
<223> Artificial

<400> 29
gataatccca tcgacagctg ctggagagga gattcgaact gggatcaaaa cagaatgaag 60

ctcgcagact gtgttgtggg atttggaagc tcgaccatgg gaggcaaagg aggagaaatt 120

tacaccgtca caagctcaga agataatcct gtgaatccta caccaggaac tttgcgctat 180

ggagcaacaa gagaaaaagc actttggata attttctctc agaatatgaa tataaagctc 240

cagatgcctt tgtatgttgc tggatataag actattgacg gcaggggagc agttgttcat 300

cttggcaatg gcggtccctg tctgtttatg aggaaagcga gccatgttat tctccatggt 360

ttgcatatac acggttgtaa tacgagtgtt ttgggggatg ttttggtaag tgagtccatt 420

ggtgtggagc ctgttcatgc tcaggatggg gacgccatta ctatgcggaa tgttacaaat 480

gcttggattg atcataattc tctctccgat tgttctgatg gtcttatcga tgttacactt 540

ggttccactg gaattactat ctccaacaat cacttcttca accatcataa agtgatgttg 600

ttaggacatg atgatacata tgatgatgac aaatctatga aagtgacagt ggccttcaat 660

caatttggac ccaatgctgg gcaaagaatg ccaagggcac gatatggact tgtacatgtt 720

gcaaacaata attatgatca atggaatata tatgctattg gtgggagttc aaatccaacc 780

attctaagtg aagggaatag tttcactgct ccaaatgaga gctacaaaaa ggaagtaaca 840

aagcgtatag ggtgtgaaac aacatcagct tgtgcgaact gggtgtggag atccacacga 900

gatgctttta ctaatggagc ttattttgta tcatcgggga aagctgaaga caccaatata 960

tacaatagta atgaagcttt caaagttgag aatgggaatg cagctcctca attaacacaa 1020

aatgctggag ttgtagcata a 1041

<210> 30
<211> 1041
<212> DNA
<213> Artificial Sequence

<220>
<223> Artificial

<400> 30
gataacccca tcgacagctg ctggcgaggc gattcaaatt gggatcaaaa tcgaatgaaa 60

ctcgcagact gcgtggtggg cttcggcagc tcaacgatgg gcggaaaagg cggcgaaatt 120

tacacggtaa ccagctcgga agataaccct gtgaacccta cccctggcac cttaaggtat 180

ggcgcaaccc gagaaaaagc attatggatc attttttcgc aaaacatgaa catcaaactc 240

caaatgcctt tatatgtggc cggctataaa accattgacg gacgaggcgc agtggtgcat 300

ttaggaaacg gaggtccctg cctattcatg cgaaaagcta gccatgtgat tctccacggt 360

ttacatatcc acggttgcaa cacaagcgtg ttaggtgatg tgttagtaag cgaatcgatt 420

ggtgtggaac ctgtgcatgc ccaagatggt gacgctatta ccatgcgaaa cgtgaccaac 480

gcctggattg atcataactc gctctcggat tgctcggatg gtttaatcga tgtgacctta 540

ggttcgaccg gcattaccat ctcgaataac cactttttta atcatcataa agtgatgtta 600

ctaggccatg atgataccta tgatgatgac aaatcgatga aagtgaccgt ggcttttaac 660

caattcggcc ccaacgccgg tcaacgaatg cctcgggcac gatatggctt agtacatgtg 720

gcaaataaca actatgatca atggaacatc tatgccattg gtggtagctc gaaccctacg 780

attctaagcg aaggtaacag ctttaccgcc cctaacgaaa gctacaaaaa agaagtaacc 840

aaaaggatcg gttgcgaaac cacctcggcc tgcgctaatt gggtgtggcg atcgacccga 900

gatgccttca ccaacggcgc ctatttcgta tcgtcaggta aagccgaaga cacgaatatc 960

tacaatagca atgaagcctt taaagtggaa aatggtaatg cagcccctca actaacccaa 1020

aatgccggcg tggtagcata a 1041

<210> 31
<211> 1131
<212> DNA
<213> Artificial Sequence

<220>
<223> Artificial

<400> 31
gctcgggctg acattggtga tgagctcgaa gcggctcaat ttaattcaac aaggaggggc 60

ttacacgaat gtgcagcaca taacataata gacaagtgtt ggaggtgcaa agctgattgg 120

gaaaaaaacc gacaagcatt agccaaatgc gcgcaaggtt ttgcaaaggg aacaactggc 180

ggattgggag gggaaatcta cgtggtgact gattgttcag atgacaatgc tgcaaatcca 240

aagccaggga cacttcgttg tggtgtcacc caagataaac ctttgtggat catctttaag 300

aaagatatgg tcataaaact taaacacgag cttgtgataa acaaagacaa gacaattgat 360

ggaagaggtg caaatgttga gatcacttgt ggcggtctca ccattcacaa cgtttgcaat 420

gtgatcattc ataacattca catacatgat attaaagtaa ccgaaggtgg aattattaag 480

gcaacggacg ctaaaccagg acatagacat aagagcgacg gagatggtat ttgtgttgct 540

ggttcttcaa agatatggat cgatcattgc acacttagtc atggtccaga tggccttatt 600

gatgtcacgt tgggtagcac agccgttacc atttccaatt gcaaatttag ccatcaccaa 660

aaaattctat tactcggagc agacaattca catgtagacg ataaaaaaat gcatgtcaca 720

gtagcattca acaggttcgc agaagcatgt gatcaaagaa tgccacgatg tcgatttgga 780

tttttccaag ttgttaacaa tgactacacc agctggggaa cgtacgccat tggtggtagt 840

gccaatccta ctatccttag ccaaggcaac cgattccatg ctccgaatga cccaatgaag 900

aaaaatgtgt tggtgagggc tgatgcacca catacagagt caatgaagtg gaattggaga 960

tctgagaaag acttgttaga aaatggagct atatttgtag catcagggtg cgacccgcat 1020

ctaaccccgg aacaaaaaag ccatttgatt ccagctgaac caggatcagc agttcttcaa 1080

ctcaccagtt gtgctggcac gctcaaatgc gttcctggaa aaccttgtta a 1131

<210> 32
<211> 1131
<212> DNA
<213> Artificial Sequence

<220>
<223> Artificial

<400> 32
gcgagggcgg acattggcga tgagcttgaa gctgcgcaat ttaattcgac gcgacgaggt 60

ctccacgaat gcgccgccca taatatcatc gacaaatgct ggcgatgcaa agcggattgg 120

gaaaaaaatc gacaagccct cgcaaaatgc gctcaaggct ttgccaaagg cacgacgggt 180

ggcttaggcg gagaaatcta cgtagtaacg gattgctcgg atgacaatgc ggccaatcct 240

aaacctggaa cgttacggtg cggcgtcacc caagataaac cgttatggat catctttaaa 300

aaagatatgg tcatcaaatt aaaacacgag ttagtaatca ataaagacaa aacgattgat 360

ggccgaggcg ccaatgtgga gatcacgtgc ggtggcctta ccattcacaa tgtgtgcaat 420

gtaatcattc ataatattca catccatgat attaaagtaa ccgaaggcgg cattattaaa 480

gccactgacg cgaaacctgg ccatcgacat aaaagtgacg gcgatggcat ttgcgtggcg 540

ggctcgtcga aaatctggat cgatcattgc acgttaagtc atggccctga tggtttaatt 600

gatgtcactt taggcagtac ggcagtgacc atttccaatt gcaaatttag tcatcaccaa 660

aaaattctac tacttggcgc cgacaattcg catgtagacg ataaaaaaat gcatgtcacg 720

gtagccttca atcgattcgc cgaagcctgc gatcaacgaa tgcctcgatg ccgatttggc 780

tttttccaag tggtgaataa tgactacacc agttggggca cttacgcaat tggcggcagt 840

gcaaatccga cgatcttaag tcaaggtaat cgattccatg cgcccaatga ccctatgaaa 900

aaaaatgtat tagtacgagc ggatgcccct catacggagt cgatgaaatg gaattggcga 960

tcggagaaag acttactcga aaatggcgcg atctttgtag cctcgggatg cgacccccat 1020

ctaacccccg aacaaaaaag tcatttaatt cctgcggaac ctggctcggc cgtgttacaa 1080

cttaccagtt gcgcgggtac tcttaaatgc gtgccgggca aaccgtgcta a 1131

<210> 33
<211> 1041
<212> DNA
<213> Artificial Sequence

<220>
<223> Artificial

<400> 33
gataatccca tcgacagctg ctggagagga gattcgaact gggatcaaaa cagaatgaag 60

ctcgcagact gtgctgtggg atttggaagc tccaccatgg gaggcaaagg aggagatttt 120

tacaccgtca caagcacaga tgataatcct gtgaatccta caccaggaac tttgcgctat 180

ggagcaacaa gagaaaaagc actttggatc attttctctc agaatatgaa tataaagctc 240

aagatgcctt tgtatgttgc tggacataag actattgacg gcaggggagc agatgttcat 300

cttggcaacg gcggtccctg tctgtttatg aggaaagtga gccatgttat tctccatagt 360

ttgcatatac acggttgtaa tacgagtgtt ttgggggatg ttttggtaag tgagtctatt 420

ggggtggagc ctgttcatgc tcaggatggg gacgccatta ctatgcgcaa tgttacaaat 480

gcttggattg atcataattc tctctccgat tgttctgatg gtcttatcga tgttacgctt 540

ggctccactg gaattactat ctccaacaat cacttcttca accatcataa agtgatgttg 600

ttaggacatg atgatacata tgacgatgac aaatctatga aagtgacagt ggcgttcaat 660

caatttggac ctaatgctgg gcaaagaatg ccaagggcac gatatggact tgtacatgtc 720

gcaaacaata attatgatcc atggaatata tatgctattg gtgggagttc aaatccaacc 780

attctaagtg aagggaatag tttcactgcc ccaagtgaga gctacaagaa ggaagtaaca 840

aagcgtatag ggtgtgaatc accatcagct tgtgcgaact gggtgtggag atctacacga 900

gatgctttta ttaatggagc ttattttgta tcatcgggga aaactgaaga gaccaatata 960

tacaatagta atgaagcttt caaagttgag aatgggaatg cagctcctca attaaccaaa 1020

aatgctggag ttgtaaccta a 1041

<210> 34
<211> 1041
<212> DNA
<213> Artificial Sequence

<220>
<223> Artificial

<400> 34
gataacccca tcgacagctg ctggcgaggc gatagtaatt gggatcaaaa tcgaatgaaa 60

ctcgccgact gcgccgttgg ctttggcagc tcgacgatgg gcggaaaagg cggcgatttt 120

tacacggtca ccagcaccga tgataacccg gttaacccga ccccgggcac cttaaggtac 180

ggcgccaccc gagagaaagc cttatggatc atttttagcc aaaacatgaa catcaaactc 240

aaaatgccgt tatacgtggc cggccataaa accattgacg gacgaggcgc cgatgtgcat 300

ttaggaaatg gaggaccctg cctatttatg cgaaaagtta gccatgtgat tctccatagc 360

ttacatatcc acggatgcaa cacgagcgtg ttaggtgatg tgttagttag cgagagcatt 420

ggtgttgagc cggtgcatgc ccaagatggt gacgctatta ccatgaggaa cgtgaccaac 480

gcctggattg atcataacag cctctcggat tgcagcgatg gattaatcga tgtgacgtta 540

ggatcgaccg gcattaccat ctcgaataac cactttttta atcatcataa agttatgtta 600

ctcggccatg atgataccta cgacgatgac aaaagcatga aagttaccgt tgcttttaac 660

caatttggcc cgaacgccgg tcaacgaatg ccgcgagccc gatacggctt agttcatgta 720

gccaataaca actacgatcc gtggaacatc tacgccattg gaggtagctc gaacccgacg 780

attctaagcg agggtaacag ctttaccgct ccgagcgaga gctacaaaaa agaggttacc 840

aaacggatcg gttgcgagtc gccgtcggcc tgcgctaatt gggtttggcg atcgacccga 900

gatgccttta ttaacggcgc ctactttgtt tcgtcaggta aaaccgagga gacgaacatc 960

tacaacagca acgaggcctt taaagtggag aacggtaacg ccgccccgca actcacgaaa 1020

aacgccggcg tggttacgtg a 1041

<210> 35
<211> 1065
<212> DNA
<213> Artificial Sequence

<220>
<223> Artificial

<400> 35
gataatccca ttgacagctg ctggagagga gatgcaaact gggatcaaaa caggatgaag 60

ctcgcagact gtgcagtggg cttcggaagc tccgccatgg gaggcaaagg aggagctttt 120

tacactgtca caagctcaga tgacgaccct gtaaatcctg caccaggtac tctgcgctac 180

ggggcaacac gagaaaggtc actgtggatc attttctcta agaatctgaa cataaagctc 240

aacatgcctt tgtacattgc tgggaataag accattgatg gcagaggagc agaagttcat 300

attggcaatg gtggtccctg tctgtttatg aggacagtga gccatgttat tctacacgga 360

ttgaatatac acggctgtaa tacaagtgtt tcggggaatg ttttgataag cgaggcttct 420

ggagtggtgc ctgttcatgc tcaggatggc gacgccatta ctatgcgcaa tgttacagat 480

gtttggattg atcataattc tctctccgat tcttctgatg gtcttgtcga tgttacactt 540

gcttccaccg gagttactat ttccaacaat cattttttca accatcataa agtgatgttg 600

ttagggcata gtgatatata tagtgatgac aaaagtatga aggtgacagt ggcattcaat 660

caatttggac ctaatgctgg acaacgaatg ccaagggcac gatatggact tatacatgtt 720

gcaaacaata attatgaccc atggagtata tatgctattg gtgggagttc aaatccaacc 780

attctaagtg aagggaatag tttcactgca ccaaatgata gcgacaagaa ggaagtaaca 840

agacgtgtag ggtgtgaatc accatcaact tgtgcaaact gggtgtggag atctacacaa 900

gattctttta ataatggagc ttattttgta tcttcaggga aaaatgaagg gactaatata 960

tacaacaata atgaagcttt caaagttgag aatgggagtg cagctcctca attaacaaaa 1020

aatgctgggg ttctaacatg cattctctct aaaccttgct catga 1065

<210> 36
<211> 1065
<212> DNA
<213> Artificial Sequence

<220>
<223> Artificial

<400> 36
gataacccca ttgactcgtg ttggcgtggt gatgccaatt gggatcagaa tcgaatgaag 60

cttgccgact gcgccgtcgg attcggttcg tcggctatgg gtggaaaagg tggtgccttt 120

tacaccgtaa cctcgtcgga tgacgaccct gtaaaccctg cccctggaac cctccgatac 180

ggagccaccc gagaacgatc gctctggata attttcagca agaacctcaa tatcaagctt 240

aatatgcctt tatacattgc cggaaacaag acgattgatg gacgtggtgc cgaagtgcat 300

attggaaacg gaggaccctg cctctttatg cgaaccgtct cgcatgtgat tctccacggt 360

ttaaacatcc acggatgcaa caccagcgtg tccggaaacg tgttaatctc ggaggccagc 420

ggtgtcgtcc ctgtgcatgc ccaagatgga gacgctatta ccatgcgaaa cgtgaccgat 480

gtgtggattg atcataacag cctttcggat agcagcgatg gattagtaga tgtgacctta 540

gcctcgacgg gtgtgaccat ttcgaataac cattttttca atcatcataa agtcatgtta 600

ttaggacata gcgatatcta tagcgatgac aaaagcatga aggtcaccgt cgccttcaac 660

cagtttggtc ctaacgccgg tcagcgaatg cctcgagccc gatatggttt aatccatgtg 720

gccaataaca actatgaccc ttggagcatc tatgccattg gaggaagcag caaccctact 780

attctcagcg aaggaaacag cttcaccgcc cctaacgatt cggacaagaa ggaagtaacc 840

cgtcgagtag gatgcgaaag ccctagcacc tgcgccaatt gggtctggcg tagcacccag 900

gatagcttta acaacggtgc ctattttgta agcagcggaa aaaacgaagg aaccaacatc 960

tacaataaca acgaagcctt caaagtggag aacggaagcg ccgcccctca gttaaccaaa 1020

aacgccggag tgctcacctg tattcttagc aaaccttgta gctga 1065

<210> 37
<211> 5
<212> PRT
<213> Artificial Sequence

<220>
<223> Artificial

<400> 37

Asp Asp Asp Asp Lys
1               5


<210> 38
<211> 4
<212> PRT
<213> Artificial Sequence

<220>
<223> Artificial

<400> 38

Ile Glu Gly Arg
1


<210> 39
<211> 4
<212> PRT
<213> Artificial Sequence

<220>
<223> Artificial

<400>  39

Ile Asp Gly Arg
1


<210>  40
<211>  6
<212>  PRT
<213>  Artificial Sequence

<220>
<223>  Artificial

<400>  40

Leu Val Pro Arg Gly Ser
1               5


<210>  41
<211>  7
<212>  PRT
<213>  Artificial Sequence

<220>
<223>  Artificial

<400>  41

Glu Asn Leu Tyr Phe Gln Gly
1               5


<210>  42
<211>  8
<212>  PRT
<213>  Artificial Sequence

<220>
<223>  Artificial

<400>  42

Leu Glu Val Leu Phe Gln Gly Pro
1               5


<210>  43
<211>  13
<212>  PRT
<213>  Artificial Sequence

<220>
<223>  Artificial

<400>  43

Asp Asn Pro Ile Asp Ser Cys Trp Arg Gly Asp Ser Asn
1               5                   10


<210>  44
<211>  10
<212>  PRT
<213>  Artificial Sequence

<220>
<223>  Artificial

<400>  44

Gly Ala Asp Asn Pro Ile Asp Ser Cys Trp
1               5                   10


<210>  45
<211>  7
<212>  PRT
<213>  Artificial Sequence

<220>
<223>  Artificial

<400>  45

Gly Pro Ile Asp Ser Cys Trp
1               5


<210>  46
<211>  8
<212>  PRT
<213>  Artificial Sequence

<220>
<223>  Artificial

<400>  46

Gly Thr Pro Ile Asp Ser Cys Trp
1               5


<210>  47
<211>  8
<212>  PRT
<213>  Artificial Sequence

<220>
<223>  Artificial

<400>  47

Gly Met Pro Ile Asp Ser Cys Trp
1               5


<210>  48
<211>  21
<212>  PRT
<213>  Artificial Sequence

<220>
<223>  Artificial

<400>  48

Lys Tyr Leu Leu Pro Thr Ala Ala Ala Gly Leu Leu Leu Leu Ala Ala
1               5                   10                  15


Gln Pro Ala Met Ala
            20


<210>  49
<211>  21
<212>  PRT
<213>  Artificial Sequence

<220>
<223>  Artificial

<400>  49

Lys Ser Leu Ile Pro Thr Ile Thr Ala Gly Leu Leu Leu Ala Leu Ser
1               5                   10                  15

Gln Pro Leu Leu Ala
            20

## Description

The present invention relates to variants of polysaccharide lyases and methods for producing the same.

Plant polysaccharide lyases comprising a beta helix structure can hardly be expressed recombinantly in a host cell (see e.g. Takai T. Int Arch Allergy Immunol (2010);153(4):431-3 and Liu Z. et al. Int Arch Allergy Immunol (2010);153(4):347-58).

In order to enhance the recombinant expression rate of such proteins several strategies may be used. For instance, the codon usage of the nucleic acid molecules may be adapted to the host cells in which the lyases may be expressed. In an alternative approach it is also possible to identify those host cells which are able to expose the proteins of interest most efficiently. However, none of these strategies seem to overcome the problem of being able to recombinantly produce plant polysaccharide lyases, including allergens such as Amb a 1 and Cup a 1, having a beta helix structure.

It is an object of the present invention to provide methods and means for producing polysaccharide lyases which comprise a beta helix structure. A further object of the present invention is to provide hypoallergenic molecules which are derived from proteins, in particular polysaccharide lyases, comprising a beta helix structure and methods for producing them.

The present invention relates therefore to a variant of a mature polysaccharide lyase having a beta helix structure, wherein said variant comprises an N- and/or C-terminal truncation and consists essentially of at least a part of the beta helix structure of the mature polysaccharide lyase.

Plant polysaccharide lyases which comprise a beta helix structure are hardly expressed by recombinant methods due to their specific three-dimensional structure. Several attempts to recombinantly produce such lyases in various hosts failed. It turned now surprisingly out that such polysaccharide lyases can be recombinantly expressed when using variants thereof which essentially consist of the beta helix structure of the wildtype polysaccharide lyases.

Some of the polysaccharide lyases known in the art exhibit allergenic activity. For instance, some members of the pectate lyase family are known to be allergens. It turned out that variants of these pectate lyase family members do not exhibit or have a reduced allergenic activity compared to the wildtype pectate layses. Such variants are considered as hypoallergenic variants of these layses.

As used herein, the term "variant consisting essentially of the beta helix structure" refers to a derivative/variant of a polysaccharide lyase which lacks essentially those parts of the wildtype polysaccharide lyase which are not associated to the beta helix structure. The phrase "consisting of" indicates that the listed elements are required or mandatory and that no other elements may be present. Thus, the phrase "consisting essentially of" indicates that the listed elements are required or mandatory, but that other elements are optional and may or may not be present. The variant according to the present invention consisting "essentially of the beta helix structure" or "essentially only the beta helix structure" means that said variant is assembled to at least 90%, preferably at least 95%, more preferably at least 98%, of the beta helix structure of the wildtype polysaccharide lyase. This means that part of the polysaccharide lyase variant may N- or C-terminally extended compared to the wildtype polysaccharide lyase.

The phrase "polysaccharide lyase" as used herein refers to a group of enzymes that cleave polysaccharide chains via a beta-elimination mechanism resulting in the formation of a double bond at the newly formed non-reducing end (see e.g. Linhardt RJ et al. Appl Biochem Biotechnol. 12(1986):135-76).

"Mature polysaccharide lyase", as used herein, refers to a polysaccharide lyase whose terminal signal sequence, if encoded by the respective cDNA, has been removed by maturation processes within the cell in which the polysaccharide lyase is naturally present.

"Beta helix structure" is a protein structure formed by the association of parallel beta strands in a helical pattern with either two or three faces. The structure is stabilized by inter-strand hydrogen bonds and protein-protein interactions (see e.g. Yoder MD et al. FASEB J. 9(1995):335-42). For identification of beta helix structures, a two step investigation can be performed using sequence as well as structure prediction software tools. In a first approach, the sequence of a given protein can be used for creation of a sequence homology alignment (e.g. via a protein blast like blastp at the NCBI). This allows the assignment of a given protein sequence to a known protein family and / or protein superfamily. In a further or alternative approach, a given protein sequence can be used for protein structure prediction (e.g. secondary structure prediction server, Dundee University, http://www.compbio.dundee.ac.uk/www-jpred/ Cole C. et al. Nucleic Acids Res (2008) 1;36(Web Server Issue):W197-201) to confirm the blast alignment and to predict secondary structure elements to assign a given protein sequence to the polysaccharide lyase families, which contain a beta-helix core element, one or both of these approaches can be applied. According to this secondary structure prediction, the first beta strand of a consecutive cluster of at least eight beta strands is to be designated as the start of the beta helix, whereas the last beta strand of a consecutive cluster of at least eight, preferably 10, more preferably 12, even more preferably 14, beta strands of this prediction is to be assigned the endpoint of the hypothetical beta helix.

The "variants", "proteins", "polypeptides" and "peptides" referred to are "isolated" "variants", "proteins", "polypeptides" and "peptides".

According to a preferred embodiment of the present invention the N- and/or C-terminal truncation comprises at least 10 amino acid residues.

As mentioned above the variant according to the present invention consists essentially of the beta helix structure of the wildtype polysaccharide lyase. Such variants comprise N- and/or C-terminal truncations of at least 10, preferably 11, 12, 13, 14, 15, 16, 17, 18, 19 or 20, more preferably 21, 22, 23, 24, 25, 26, 27, 28, 29 or 30, even more preferably 31, 32, 33, 34, 35, 36, 37, 38, 39, 40, 41, 42, 43, 44, 45, 46, 47, 48, 49 or 50, amino acid residues in respect to the wildtype polysaccharide lyase. Of course the number of truncated amino acid residues may vary between the N- and C-terminus of the polysaccharide lyase and may depend on the at which amino acid position within the polysaccharide lyase the beta helix structure begins or ends, respectively.

According to a particularly preferred embodiment of the present invention said N-terminal truncation comprises the region extending from the N-terminal end of the polysaccharide lyase to approximately 1 to 50, preferably 1 to 40, more preferably 1 to 30, even more preferably 1 to 20, particularly 1 to 10, amino acid residues before the N-terminus of the first beta sheet being part of the beta helix structure and said C-terminal truncation comprises the region extending from the C-terminal end of the polysaccharide lyase to approximately 1 to 50, preferably 1 to 40, more preferably 1 to 30, even more preferably 1 to 20, particularly 1 to 10, amino acid residues after the C-terminus of the last beta sheet part of the beta helix structure, under the provisio that the first and last beta sheet of the beta helix structure remains intact.

The variant according to the present invention may comprise an N-terminal truncation which includes the region of the polysaccharide lyase from the N-terminal end to the first amino acid residue being part of the first beta sheet being part of the beta helix structure. The C-terminal truncation may include the region of the polysaccharide lyase from the C-terminal end to the last amino acid residue of the last beta sheet being part of the beta helix structure. Consequently the N- and/or C-terminal truncation may involve 1 to 50, 1 to 49, 1 to 48, 1 to 47, 1 to 46, 1 to 45, 1 to 44, 1 to 43, 1 to 42, 1 to 41, 1 to 40, 1 to 39, 1 to 38, 1 to 37, 1 to 36, 1 to 35, 1 to 34, 1 to 33, 1 to 32, 1 to 31, 1 to 30, 1 to 29, 1 to 28, 1 to 27, 1 to 26, 1 to 25, 1 to 24, 1 to 23, 1 to 22, 1 to 21, 1 to 20, 1 to 19, 1 to 18, 1 to 17, 1 to 16, 1 to 15, 1 to 14, 1 to 13, 1 to 12, 1 to 11, 1 to 10, 1 to 9, 1 to 8, 1 to 7, 1 to 6, 1 to 5, 1 to 4, 1 to 3 or 1 to 2.

According to a preferred embodiment of the present invention the polysaccharide lyase is a member of a family of proteins selected from the group consisting of pectate lyase (EC 4.2.2.2), hyaluronate lyase (EC 4.2.2.1), poly(β-D-mannuronate) lyase (EC 4.2.2.3), chondroitin AC lyase (EC 4.2.2.5), oligogalacturonide lyase (EC 4.2.2.6), heparin lyase (EC 4.2.2.7), heparin-sulfate lyase (EC 4.2.2.8), pectate disaccharide-lyase (EC 4.2.2.9), pectin lyase (EC 4.2.2.10), poly(α-L-guluronate) lyase (EC 4.2.2.11), xanthan lyase (EC 4.2.2.12), exo-(1-->4)-α-D-glucan lyase (EC 4.2.2.13), glucuronan lyase (EC 4.2.2.14), anhydrosialidase (EC 4.2.2.15), levan fructotransferase (EC 4.2.2.16), inulin fructotransferase (EC 4.2.2.17, EC 4.2.2.18), chondroitin B lyase (EC 4.2.2.19), chondroitin-sulfate-ABC endolyase (EC 4.2.2.20), chondroitin-sulfate-ABC exolyase (EC 4.2.2.21) and pectate trisaccharide-lyase (EC 4.2.2.22).

As mentioned above some allergens are polysaccharide lyases, in particular pectate lyases. Therefore the variant according to the present invention being derived from a polysaccharide lyase is preferably an allergenic pectate lyase, preferably derived from Chamaecyparis obtusa (Japanese cypress), Cryptomeria japonica (Sugi), Cupressus arizonica (Cypress), Juniperus ashei (Mountain cedar), Artemisia vulgaris (Mugwort) and Ambrosia artemisiifolia (Short ragweed).

According to a preferred embodiment of the present invention the allergen is selected from the group consisting of Amb a 1, , Art v 6, Cha o 1, Cup a 1, Cry j 1 and Jun a 1.

It turned surprisingly out that a variant of an allergen consisting essentially of the beta helix structure as defined herein exhibits a reduced IgE reactivity compared to the wildtype allergen. Therefore the variant according to the present invention derived from an allergen is to be considered as a hypoallergenic molecule.

The variant according to the present invention derived from Amb a 1 consists essentially of amino acid sequence SEQ ID No: 1, the variant derived from Cry j 1 consists essentially of amino acid sequence SEQ ID No: 2, the variant derived from Cup a 1 consists essentially of amino acid sequence SEQ ID No: 3, the variant derived from Jun a 1 consists essentially of amino acid sequence SEQ ID No: 4, the variant derived from Cha o 1 consists essentially of amino acid sequence SEQ ID No: 5 and the variant derived from Art v 6 consists essentially of amino acid sequence SEQ ID No: 6.

Another aspect of the present invention relates to a polypeptide comprising a secretion signal sequence fused directly or via a linker to a variant according to the present invention as defined above or to a mature polysaccharide lyase as defined herein or a fragment thereof, namely a pectate lyase, hyaluronate lyase, poly(β-D-mannuronate) lyase, chondroitin AC lyase, oligogalacturonide lyase, heparin lyase, heparin-sulfate lyase, pectate disaccharide-lyase, pectin lyase, poly(α-L-guluronate) lyase, xanthan lyase, exo-(1-->4)-α-D-glucan lyase, glucuronan lyase, anhydrosialidase, levan fructotransferase, inulin fructotransferase, chondroitin B lyase, chondroitin-sulfate-ABC endolyase, chondroitin-sulfate-ABC exolyase and pectate trisaccharide-lyase, preferably selected from the group consisting of Amb a 1, , Art v 6, Cha o 1, Cup a 1, Cry j 1 and Jun a 1.

It turned surprisingly out that polysaccharide lyases and variants thereof as defined in the present invention show a higher stability and can be recombinantly expressed in higher yields. In order to avoid the removal of the secretion signal sequence within a host cell it is particularly advantageous to use heterologous secretion signal sequences and/or to introduce a linker between the secretion signal sequence and the variant or the mature polysaccharide lyase of the present invention which does not contain a naturally occurring protease cleavage site. In order to obtain a protein product without the secretion signal sequence it is advantageous to provide a linker which comprises an artificial protease cleavage site.

Secretion signal sequences or peptides are useful in directing the secretion of the polypeptides of the present invention. In certain embodiments, the polypeptide may comprise its own signal peptide, i.e., a "native" signal peptide. In other embodiments, the polypeptide may be fused to a "heterologous" secretion signal sequence/peptide, i.e., a signal peptide that is derived from a protein that is different from the wild-type polypeptide. According to a preferred embodiment of the present invention the secretion signal sequence is a bacterial, fungal or eukaryotic, in particular mammalian, origin.

In a particularly preferred embodiment of the present invention the secretion signal sequences are bacterial signal peptides. Bacterial secretion signal sequences differ from each other only in minor aspects. For instance, signal sequences of gram-positive bacteria typically have a slightly longer and more basic N-terminus than signal peptides from other bacteria. Alternatively eukaryotic secretion signal sequences/peptides may be employed. For example, it has been shown that the signal peptides of E. coli periplasmic and outer membrane proteins, of vacuolar and secreted protein of Saccharomyces cerevisiae, and of constitutively secreted and storage granule proteins of multi-cellular eukaryotes can be exchanged without affecting the targeting of the mature protein. Indeed, in certain non-naturally occurring environments (e.g. expression of eukaryotic genes in bacterial cells), secretion signal sequences/peptides of some proteins have been shown to mediate efficient secretion. For instance, insulin and ovalbumin are secreted by E. coli upon expression of these genes, and yeast is able to efficiently secrete various secretory proteins derived from multicellular eukaryotes.

According to a preferred embodiment of the present invention the secretion signal sequence is selected from the group consisting of a PelB, a OmpA, a StII, an EX, a PhoA, a OmpF, a PhoE, a MalE, an OmpC, a LPP, a LamB, an OmpT, a LTB, a phosphatase (pho1) and an invertase (Suc) secretion signal sequence and an α-mating factor prepro-sequence.

Secretion signal sequences/peptides may be derived from extracellular bacterial proteins. Examples of signal peptides or signal sequences that may be incorporated into the polypeptide of the present invention include Bacillus subtilis PgsA signal sequence, E. coli OmpA signal sequence, E. coli Ag43 signal sequence, B. licheniformis penicillinase signal sequence, E. coli lipoprotein (lpp) signal sequence, Bacillus cereus penicillinase III signal sequence, S. aureus penicillinase signal sequence, Neisseria IgA protease signal sequence, Serratia serine proteins signal sequence, Pseudomonas endoglucanase signal sequence, E. coli STA enterotoxin signal sequence, Klebsiella or E. coli cloacin (colicin) signal sequence, Proteus or Serratia Shla/HpmA hemolysin signal sequence, Klebsiella pullalanase signal sequence, Pseudomonas, Erwinia, or Xanthomonas exoenzyme signal sequences, Aeromonas aerolysin signal sequence, Vibrio CTX toxin signal sequence, Pseudomonas or Neisseria Type IV pillin signal sequence, E. coli P-type pilin signal sequence, E. coli Type-I pilin signal sequence, Yersinia OM proteins (YOPS) signal sequences, E. coli or Caulobacter flagellin signal sequences, Shigella or Salmonella Invasion protein signal sequences, E. coli, Proteus, or Morganella HlyA hemolysin signal sequences, Bordatella pertussis CyaA cyclolysin signal sequence, Erwinia, Serratia, or Proteus protease signal sequences, Rhizobium NodO signal sequence, E. coli ColV colicin signal sequence, among others known in the art.

These or other signal peptide sequences can be identified according to routine techniques in the art. For instance, PrediSi (Prediction of Signal peptides) represents one exemplary tool for predicting signal peptide sequences and their cleavage positions in both bacterial and eukaryotic amino acid sequences (see, e.g., Hiller et al, Nucleic Acids Research. 32:W375-W379, 2004). This software tool is especially useful for the analysis of large datasets in real time with high accuracy, and is based on a position weight matrix approach improved by a frequency correction that takes in to consideration the amino acid bias present in proteins. Also available is a Hidden Markov Model method for the prediction of lipoprotein signal peptides of Gram-positive bacteria, trained on a set of 67 experimentally verified lipoproteins (see, e.g., Bagos et al, J. Proteome Res. 7:5082-5093, 2008).

The polypeptides of the present invention is used to a secretion signal sequence may be prepared using standard techniques. For example, DNA sequences encoding the polypeptide components of a desired fusion may be assembled separately, and ligated into an appropriate expression vector. The 3' end of the DNA sequence encoding one polypeptide component can be ligated, with or without a peptide linker, to the 5' end of a DNA sequence encoding the second polypeptide component so that the reading frames of the sequences are in phase. This permits translation into a single fusion protein that retains the biological activity of both component polypeptides.

According to a particularly preferred embodiment of the present invention peptide linker sequences may be employed to separate the variant of the present invention from the secretion signal sequence. Such peptide linker sequences may be incorporated into the fusion protein using standard techniques well known in the art. The peptide linker sequence incorporated between the variant of the present invention and the secretion signal sequence comprises advantageously a protease cleavage site. The presence of one or more protease cleavage sites allows to remove from the variant of the present invention the secretion signal sequence either by the expression host itself or by the addition of a respective protease after the isolation of the polypeptide of the present invention from the cultivation medium in which the polypeptide of the present invention is secreted in the course of its recombinant production.

According to a particularly preferred embodiment of the present invention the protease cleavage site is capable of being cleaved by thrombin, chymotrypsin, trypsin, pepsin, subtilisin, enterokinase, factor Xa, TEV protease and PreScission protease, whereby TEV protease is particularly preferred.

The sequence encoding a protease cleavage site can be included between the secretion signal or leader sequence or the affinity tag and the variant, protein or polypeptide of the present invention, thus permitting - as mentioned before - the removal of these sequences after incubation with a specific protease. Suitable protease cleavage sites for incorporation into the polypeptides of the invention include enterokinase (cleavage site Asp-Asp-Asp-Asp-Lys) (SEQ ID NO: 37), factor Xa (cleavage site Ile-Glu-Gly-Arg (SEQ ID NO: 38) or lle-Asp-GIy-Arg (SEQ ID NO: 39)), thrombin (cleavage site Leu-Val-Pro-Arg-Gly-Ser; Arg-Gly) (SEQ ID NO: 40), TEV protease (cleavage site Glu-Asn-Leu-Tyr-Phe-Gln-Gly) (SEQ ID NO: 41), PreScission protease (cleavage site Leu-Glu-Val-Leu-Phe-Gln-Gly-Pro) (SEQ ID NO: 42), inteins (self-splicing intein sequences that can be activated, e.g., by adjustments to pH (Chong et al., Gene, 792 (1997):27-281))and the like.

In an alternative embodiment of the present invention the cleavage site may be a chemical cleavage site including tryptophan residues cleaved by 3-bromo-3-methyl-2-(2- nitrophenylmercapto)-3H-indole; cysteine residues cleaved by 2-nitroso-5-thiocyano benzoic acid; the dipeptides -Asp-Pro- or -Asn-Gly- which can be cleaved by acid and hydroxylamine, respectively; and a methionine residue which is specifically cleaved by cyanogen bromide (CNBr) .

The linker sequence may generally be from 1 to 50, preferably 1 to 40, more preferably 1 to 30, even more preferably 1 to 20, most preferably 1 to 10 amino acids in length. Linker sequences are typically not required when the fusion of the variant of the present invention to the secretion signal sequence results in a protease cleavage site.

The variants, proteins and polypeptides of the present invention can also be fused directly or via a linker (which may be a protease cleavage site) to a tag which is employed for purification or for detection. In a particular embodiment, the variant, protein or polypeptide of the present invention comprises the glutathione-S-transferase protein tag which provides the basis for rapid high-affinity purification of the polypeptide of the invention. Indeed, such a GST-fusion protein can then be purified from cells via its high affinity for glutathione. Agarose beads can be coupled to glutathione and such glutathione-agarose beads bind GST-proteins. Thus, in a particular embodiment of the invention, the variant, protein or polypeptide of the invention is bound to a solid support. In a preferred embodiment, if the polypeptide of the invention comprises a GST moiety, the polypeptide is coupled to a glutathione-modified support. In a particular case, the glutathione modified support is a glutathione-agarose bead.

Another aspect of the present invention relates to a nucleic acid molecule encoding a variant of the polysaccharide lyase or a polypeptide according to the present invention.

According to a preferred embodiment of the present invention to nucleic acid molecule comprises a nucleotide sequence encoding for a leader sequence.

The nucleotide sequence encoding for a leader sequence which is provided at the 5'-end of and in-frame with the nucleotide sequence encoding the variant of the present invention allows once transcribed and translated to direct the secretion of the translated polypeptide out from a host cell. The leader sequence may be of any origin provided that the host cell in which the polypeptide is expressed is capable to recognise the respective secretion signal sequences.

The leader sequence is preferably selected from the group consisting of PelB and PelC.

The nucleic acid molecule according to the present invention can of course comprise further nucleotide sequences encoding for polypeptides and peptides which may facilitate the purification, enrichment or stabilisation of the variant encoded.

According to particular preferred embodiment of the present invention the nucleotide sequence of said nucleic acid molecule is codon optimised and/or codon harmonised.

Optimizing codons to achieve high levels of protein expression in host cells is an often used method. Such methods regularly comprise the identification of "non-preferred" or a "less preferred" codon and the replacement of one or more of these non-preferred or less preferred codons with a "preferred codon" encoding the same amino acid as the replaced codon and at least one non- preferred or less preferred codon in the nucleic acid has been replaced by a preferred codon encoding the same amino acid. A preferred codon is a codon over-represented in coding sequences in genes in the host cell and a non-preferred or less preferred codon is a codon under-represented in coding sequences in genes in the host cell.

In order to stabilise the variants of the present invention within the cell the N-terminal amino acid residues, preferably the first two residues or at least the second, are preferably exchanged by respective stabilising amino acid residues (e.g. glycine) (see e.g. Varshavsky A. Nature Structural & Molecular Biology 15(2008):1238-40; Dougan DA et al. Molecular Microbiology 76(2010): 545-558; Tobias JW, et al. Science 254(1991):1374-7) .

Host cells for expressing the nucleic acids, expression cassettes and vectors of the invention include bacteria, yeast, fungi, plant cells, insect cells and mammalian cells. Thus, the invention provides methods for optimizing codon usage in all of these cells, codon-altered nucleic acids and polypeptides made by the codon-altered nucleic acids. Exemplary host cells include gram negative bacteria, such as Escherichia coli and Pseudomonas fluorescens; gram positive bacteria, such as Lactobacillus gas-seri, Lactococcus lactis, Lactococcus cremoris, Bacillus subtilis. Exemplary host cells also include eukaryotic organisms, e.g., various yeast, such as Sacchammyces Sp., including Saccharomyces cerevisiae, Schizosaccharomyces pombe, Pichia pastoris and Kluyveromyces lactis, Hansenula polymorpha, Aspergillus niger, and mammalian cells and cell lines and insect cells and cell lines. Thus, the invention also includes nucleic acids and polypeptides optimized for expression in these organisms and species. It is particularly preferred to use methods in which the codons are harmonised. Methods for harmonising codons are well known in the art (Angov E, et al. PloSOne 3(2008):e2189). In such methods the codon usage of the host cell as well as of the cell from which the nucleic acid molecule encoding the polypeptide to be transcribed and translated is derived from is considered. This allows to obtain a nucleotide sequence whose codon usage is balanced between the host cell and the cell originating the nucleic acid molecule encoding the polypeptide to be expressed.

The codon usage for a given organism is available at the codon usage database http://www.kazusa.or.jp/codon/ (Nakamura Y. et al. Nucleic Acids Res (2000);28(1):292). The codon usage of pectate lyase genes was determined for the respective source organism. The codon usage of the host organism was used to transfer the codon usage for the gene of interest from the source organism to the host organism. Therefore, E. coli codons, for instance, encoding the respective amino acid at a given position and showing the codon usage, which was closest to the original codon of the source organism for the respective amino acid, were selected. The DNA code was then changed accordingly and the resulting gene with new codon composition was generated synthetically.

According to a preferred embodiment of the present invention the nucleic acid molecule encoding the variant of Amb a 1 has the nucleotide sequence SEQ ID No: 7 or SEQ ID No: 8, the nucleic acid molecule encoding Cry j 1 has the nucleotide sequence SEQ ID No: 9 or 10, the nucleic acid molecule encoding Cup a 1 has the nucleotide sequence SEQ ID No: 11 or 12, the nucleic acid molecule encoding Art v 6 has the nucleotide sequence SEQ ID No: 13 or 14, the nucleic acid molecule encoding Jun a 1 has the nucleotide sequence SEQ ID No: 15 or 16, and the nucleic acid molecule encoding Cha o 1 has the nucleotide sequence SEQ ID No: 17 or 18.

Another aspect of the present invention relates to a nucleic acid molecule having or comprising nucleic acid sequence SEQ ID No. 7, SEQ ID No. 8, SEQ ID No. 9, SEQ ID No. 10, SEQ ID No. 11, SEQ ID No. 12, SEQ ID No. 13, SEQ ID No. 14, SEQ ID No. 15, SEQ ID No. 16, SEQ ID No. 17, SEQ ID No. 18, SEQ ID No. 25, SEQ ID No. 26, SEQ ID No. 27, SEQ ID No. 28, SEQ ID No. 29, SEQ ID No. 30, SEQ ID No. 31, SEQ ID No. 32, SEQ ID No. 33, SEQ ID No. 34, SEQ ID No. 35 or SEQ ID No. 36. These nucleic acid molecules may fused to nucleic acid sequences encoding a secretion signal sequence as described herein. Also encompassed by the present invention are the polypeptides encoded by these nucleic acid molecules.

Another aspect of the present invention relates to vector comprising a nucleic acid molecule according to the present invention.

The nucleic acid molecule of the present invention encoding the variant of the present invention may be operably linked to transcriptional and translational control sequences, e.g. promoters or enhancers, to direct or modulate RNA synthesis and expression. The expression control sequence can be in an expression vector. Exemplary bacterial promoters include lad, lacZ, T3, T7, gpt, lambda PR, PL and trp. Exemplary eukaryotic promoters include CMV immediate early, HSV thymidine kinase, early and late SV40, LTRs from retrovirus, and mouse metallothionein I.

Promoters suitable for expressing, or over-expressing, a polypeptide in bacteria include the E. coli lac or trp promoters, the lad promoter, the lacZ promoter, the T3 promoter, the T7 promoter, the gpt promoter, the lambda PR promoter, the lambda PL promoter, promoters from operons encoding glycolytic enzymes such as 3-phosphoglycerate kinase (PGK), and the acid phosphatase promoter. Eukaryotic promoters include the CMV immediate early promoter, the HSV thymidine kinase promoter, heat shock promoters, the early and late SV40 promoter, LTRs from retroviruses, and the mouse metallothionein-I promoter. Other promoters known to control expression of genes in prokaryotic or eukaryotic cells or their viruses may also be used. The present invention relates also to expression systems such as expression cassettes, vectors, cloning vehicles and the like, comprising nucleic acids of the invention, e.g., sequences encoding the variants of the invention, for expression, and over-expression, of the polypeptides of the invention. Expression vectors and cloning vehicles of the invention can comprise viral particles, baculovirus, phage, plasmids, phagemids, cosmids, fosmids, bacterial artificial chromosomes, viral DNA (e.g., vaccinia, adenovirus, foul pox virus, pseudorabies and derivatives of SV40), Pl - based artificial chromosomes, yeast plasmids, yeast artificial chromosomes, and any other vectors specific for specific hosts of interest (such as bacillus, Aspergillus and yeast). Exemplary bacterial vectors include pQE vectors (Qiagen), pBluescript plasmids, pNH vectors, (lambda-ZAP vectors (Stratagene); ptrc99a, pKK223-3, pDR540, pRIT2T (Pharmacia); Eukaryotic: pXTl, pSG5 (Stratagene), pSVK3, pBPV, pMSG, pSVLSV40 (Pharmacia). However, any other plasmid or other vector may be used so long as they are replicable and viable in the host. Low copy number or high copy number vectors may be employed with the present invention.

As representative examples of expression vectors which may be used there may be mentioned viral particles, baculovirus, phage, plasmids, phagemids, cosmids, fosmids, bacterial artificial chromosomes, viral DNA (e.g., vaccinia, adenovirus, foul pox virus, pseudorabies and derivatives of SV40), Pl -based artificial chromosomes, yeast plasmids, yeast artificial chromosomes, and any other vectors specific for specific hosts of interest (such as bacillus, Aspergillus and yeast). Thus, for example, the DNA may be included in any one of a variety of expression vectors for expressing a polypeptide. Such vectors include chromosomal, nonchromosomal and synthetic DNA sequences. Large numbers of suitable vectors are known to those of skill in the art.

The nucleic acid sequence in the expression vector is operatively linked to an appropriate expression control sequence(s) (promoter) to direct RNA synthesis. Particular named bacterial promoters include lad, lacZ, T3, T7, gpt, lambda PR, PL and trp. Eukaryotic promoters include CMV immediate early, HSV thymidine kinase, early and late SV40, LTRs from retrovirus, and mouse metallothionein-I. Selection of the appropriate vector and promoter is well within the level of ordinary skill in the art. The expression vector also contains a ribosome binding site for translation initiation and a transcription terminator. The vector may also include appropriate sequences for amplifying expression. Promoter regions can be selected from any desired gene using CAT (chloramphenicol transferase) vectors or other vectors with selectable markers. In addition, the expression vectors can contain one or more selectable marker genes to provide a phenotypic trait for selection of transformed host cells such as dihydrofolate reductase or neomycin resistance for eukaryotic cell culture, or tetracycline, ampicillin, or kanamycin resistance in E. coli.

In one aspect, expression cassettes of the invention comprise a sequence of the invention and a nucleotide sequence which is capable of affecting expression of a structural gene (i.e., a protein coding sequence) in a host compatible with such sequences. Expression cassettes include at least a promoter operably linked with the polypeptide coding sequence; and, optionally, with other sequences, e.g. transcription termination signals. Additional factors necessary or helpful in effecting expression may also be used, e.g., enhancers. In one aspect, "operably linked" as used herein refers to linkage of a promoter upstream from a DNA sequence such that the promoter mediates transcription of the DNA sequence. Thus, expression cassettes also include plasmids, expression vectors, recombinant viruses, any form of recombinant naked DNA vector, and the like. In one aspect, a "vector" comprises a nucleic acid that can infect, transfect, transiently or permanently transduce a cell. A vector can be a naked nucleic acid, or a nucleic acid complexed with protein or lipid.

A DNA sequence may be inserted into a vector by a variety of procedures. In general, the DNA sequence is ligated to the desired position in the vector following digestion of the insert and the vector with appropriate restriction endonucleases. Alternatively, blunt ends in both the insert and the vector may be ligated. A variety of cloning techniques are known in the art.

The invention relates also to transformed cells comprising a nucleic acid sequence according to the present invention, e.g., a sequence encoding a variant of a polysaccharide lyase according to the invention, or comprising an expression cassette, vector, cloning vehicle, expression vector, or cloning vector of the invention. The host cell may be any of the host cells familiar to those skilled in the art, including prokaryotic cells, eukaryotic cells, such as bacterial cells, fungal cells, yeast cells, mammalian cells, insect cells, or plant cells. Exemplary bacterial cells include any species within the genera Escherichia, Bacillus, Streptomyces, Salmonella, Pseudomonas, Lactococcus, and Staphylococcus, including, e.g., Escherichia coli, Lactococcus lactis, Bacillus subtilis, Bacillus cereus, Salmonella typhimurium, Pseudomonas fluorescens . Exemplary fungal cells include any species of Aspergillus, including Aspergillus niger. Exemplary yeast cells include any species of Pichia, Saccharomyces, Schizosacchammyces, or Schwanniomyces, including Pichia pastoris, Saccharomyces cerevisiae, or Schizosaccharomyces pombe. Exemplary insect cells include any species of Spodoptera or Drosophila, including Drosophila S2 and Spodoptera S/P.Exemplary insect cells include Drosophila S2 and Spodoptera Sf9. Exemplary animal cells include CHO, COS or Bowes melanoma or any mouse or human cell line. The selection of an appropriate host is within the abilities of those skilled in the art.

The vector may be introduced into the host cells using any of a variety of techniques, including transformation, transfection, transduction, viral infection, gene guns, or Ti-mediated gene transfer. Particular methods include calcium phosphate transfection, DEAE-Dextran mediated transfection, lipofection, or electroporation.

Where appropriate, the engineered host cells can be cultured in conventional nutrient media modified as appropriate for activating promoters, selecting transformants or amplifying the genes of the invention. Following transformation of a suitable host strain and growth of the host strain to an appropriate cell density, the selected promoter may be induced by appropriate means (e.g., temperature shift or chemical induction) and the cells may be cultured for an additional period to allow them to produce the desired polypeptide or fragment thereof.

The expressed and secreted variants can be recovered and purified from recombinant cell cultures by methods including ammonium sulfate or ethanol precipitation, acid extraction, anion or cation exchange chromatography, phosphocellulose chromatography, hydrophobic interaction chromatography, affinity chromatography, hydroxylapatite chromatography and lectin chromatography.

The constructs in host cells can be used in a conventional manner to produce the gene product encoded by the recombinant sequence. Depending upon the host employed in a recombinant production procedure, the polypeptides produced by host cells containing the vector may be glycosylated or may be non-glycosylated. Polypeptides of the invention may or may not also include an initial methionine amino acid residue.

Cell-free translation systems can also be employed to produce a polypeptide of the invention. Cell-free translation systems can use mRNAs transcribed from a nucleic acid construct comprising a promoter operably linked to a nucleic acid encoding the polypeptide or fragment thereof. In some aspects, the nucleic acid construct may be linearized prior to conducting an in vitro transcription reaction. The transcribed mRNA is then incubated with an appropriate cell-free translation extract, such as a rabbit reticulocyte extract, to produce the desired polypeptide or fragment thereof.

Host cells comprising the polynucleotides of interest can be cultured in conventional nutrient media modified as appropriate for activating promoters, selecting transformants or amplifying genes. The culture conditions, such as temperature, pH and the like, are those previously used with the host cell selected for expression, and will be apparent to the ordinarily skilled artisan. The clones which are identified as having the specified enzyme activity may then be sequenced to identify the polynucleotide sequence encoding an enzyme having the enhanced activity.

Yet another aspect of the present invention relates to a method for the recombinant production of a polysaccharide lyase variant having a beta helix structure according to the present invention comprising the steps of:
a) cultivating a host cell comprising a nucleic acid mole cule or a vector according to the present invention in a culture medium,
b) isolating the polysaccharide lyase variant from the supernatant of the culture medium.

According to a preferred embodiment of the present invention the secretion signal sequence is removed from the polysaccharide lyase variant or polypeptide by incubating the lyase variant or polypeptide of step b) to a protease capable to cleave the secretion signal sequence from the lyase variant of a polypeptide. Respective methods are well known to the person skilled in the art.

Another aspect of the present invention relates to a method for identifying a variant according to the present invention, wherein the N- and/or C-terminal truncation of the polysaccharide lyase is identified by
(i) determining the two and three dimensional structure of the polysaccharide lyase,
(ii) identifying the beta helix structure,
(iii) determining the first and last beta sheet being part of the beta helix structure,
(iv) truncating the region extending from the N-terminal end of the polysaccharide lyase to approximately 1 to 50 amino acid residues before the N-terminus of the first beta sheet being part of the beta helix structure of said variant and the region extending from the C-terminal end of the polysaccharide lyase to approximately 1 to 50 amino acid residues after the C-terminus of the last beta sheet part of the beta helix structure, under the provisio that the first and last beta sheet of the beta helix structure remains intact.

The single methods identified above are well known in the art.

The present invention relates also to the following embodiments:
1. A variant of a mature polysaccharide lyase having a beta helix structure, wherein said variant comprises an N- and/or C-terminal truncation and consists essentially of at least a part of the beta helix structure of the mature polysaccharide lyase.
2. Variant according to embodiment 1, wherein the N- and/or C-terminal truncation comprises at least 10 amino acid residues.
3. Variant according to embodiment 1 or 2, wherein said N-terminal truncation comprises the region extending from the N-terminal end of the polysaccharide lyase to approximately 1 to 50 amino acid residues before the N-terminus of the first beta sheet being part of the beta helix structure and said C-terminal truncation comprises the region extending from the C-terminal end of the polysaccharide lyase to approximately 1 to 50 amino acid residues after the C-terminus of the last beta sheet part of the beta helix structure, under the provisio that the first and last beta sheet of the beta helix structure remains intact.
4. Variant according to any one of embodiments 1 to 3, wherein the polysaccharide lyase is a member of a family of proteins selected from the group consisting of pectate lyase, hyaluronate lyase, poly (β-D-mannuronate) lyase, chondroitin AC lyase, oligogalacturonide lyase, heparin lyase, heparin-sulfate lyase, pectate disaccharide-lyase, pectin lyase, poly (α-L-guluronate) lyase, xanthan lyase, exo-(1-->4)-α-D-glucan lyase, glucuronan lyase, anhydrosialidase, levan fructotransferase, inulin fructotransferase, chondroitin B lyase, chondroitin-sulfate-ABC endolyase, chondroitin-sulfate-ABC exolyase and pectate trisaccharide-lyase.
5. Variant according to embodiment 4, wherein the polysaccharide lyase is a pectate lyase, preferably an allergenic pectate lyase.
6. Variant according to embodiment 5, wherein the allergen is selected from the group consisting of Amb a 1, , Art v 6, Cha o 1, Cup a 1, Cry j 1 and Jun a 1.
7. Variant according to embodiment 5 or 6, wherein the variant is hypoallergenic.
8. Variant according to embodiment 7, wherein the variant derived from Amb a 1 consists essentially of amino acid sequence SEQ ID No: 1, the variant derived from Cry j 1 consists essentially of amino acid sequence SEQ ID No: 2, the variant derived from Cup a 1 consists essentially of amino acid sequence SEQ ID No: 3, the variant derived from Jun a 1 consists essentially of amino acid sequence SEQ ID No: 4, the variant derived from Cha o 1 consists essentially of amino acid sequence SEQ ID No: 5 and the variant derived from Art v 6 consists essentially of amino acid sequence SEQ ID No: 6.
9. Polypeptide comprising a secretion signal sequence fused directly or via a linker to a variant according to any one of embodiments 1 to 8 or to a mature polysaccharide lyase as defined in any one of embodiments 4 to 6 or a fragment thereof.
10. Polypeptide according to embodiment 9, wherein the secretion signal sequence is of bacterial, fungal or mammalian origin.
11. Polypeptide according to embodiment 9 or 10, wherein the secretion signal sequence is selected from the group consisting of a PelB, a OmpA, a StII, an EX, a PhoA, a OmpF, a PhoE, a MalE, an OmpC, a LPP, a LamB, an OmpT, a LTB, a phosphatase (pho1) and an invertase (Suc) secretion signal sequence and an α-mating factor prepro-sequence.
12. Polypeptide according to any one of embodiments 9 to 11, wherein the linker comprises a protease cleavage site.
13. Polypeptide according to embodiment 12, wherein the protease cleavage site is capable of being cleaved by thrombin, chymotrypsin, trypsin, pepsin, subtilisin, enterokinase, factor Xa, TEV protease and PreScission protease, whereby TEV protease is particularly preferred.
14. Nucleic acid molecule encoding a variant of a polysaccharide lyase according to any one of embodiments 1 to 8 or a polypeptide according to any one of embodiments 9 to 13.
15. Nucleic acid molecule according to embodiment 14, wherein said nucleic acid molecule comprises further a nucleotide sequence encoding for a leader sequence.
16. Nucleic acid molecule according to embodiment 15, wherein the leader sequence is selected from the group consisting of PelB and PelC.
17. Nucleic acid molecule according to any one of embodiments 14 to 16, wherein the nucleotide sequence of said nucleic acid molecule is codon optimised and/or codon harmonised.
18. Nucleic acid molecule according to any one of embodiments 14 to 17, wherein the nucleic acid molecule encoding the variant of Amb a 1 has the nucleotide sequence SEQ ID No: 7 or SEQ ID No: 8, the nucleic acid molecule encoding the variant of Cry j 1 has the nucleotide sequence SEQ ID No: 9 or 10, the nucleic acid molecule encoding the variant of Cup a 1 has the nucleotide sequence SEQ ID No: 11 or 12, the nucleic acid molecule encoding the variant of Art v 6 has the nucleotide sequence SEQ ID No: 13 or 14, the nucleic acid molecule encoding the variant of Jun a 1 has the nucleotide sequence SEQ ID No: 15 or 16 and the nucleic acid molecule encoding Cha o 1 has the nucleotide sequence SEQ ID No: 17 or 18.
19. Vector comprising a nucleic acid molecule according to any one of embodiments 14 to 18.
20. Method for the recombinant production of a polysaccharide lyase variant having a beta helix structure according to any one of embodiments 1 to 8 or a polypeptide according to any one of embodiments 9 to 13 comprising the steps of:
   a) cultivating a host cell comprising a nucleic acid molecule according to any one of embodiments 14 to 18 or a vector according to embodiment 19 in a culture medium,
   b) isolating the polysaccharide lyase variant or polypeptide from the supernatant of the culture medium.
21. Method according to embodiment 20, wherein the secretion signal sequence is removed from the polysaccharide lyase variant or polypeptide by incubating the lyase variant or polypeptide of step b) to a protease capable to cleave the secretion signal sequence from the lyase variant of a polypeptide.

The present invention is further illustrated in the following figures and examples, however, without being restricted thereto.
Fig. 1 shows Cry j 1 with modified amino termini. (1) with additional Gly-Ala. (2) deletion of Asp-Asn, instead Gly. (3) exchange Asp-Asn to Gly-Thr. (4) exchange Asp-Asn to Gly-Met.
Fig. 2 shows a soluble bacterial protein fraction after expression in E.coli. Coomassie-stained SDS-PAGE and Wetsern Blot using a murine monoclonal anti Cry j 1 antibody.
Fig. 3 shows the differences in N-terminal codon usage after codon optimization and codon harmonization of Cry j 1, including additional Gly-Ala at the amino terminus.
Fig. 4 shows SDS-PAGE and Western Blot using murine anti Cry j 1 monoclonal detection antibody of Cry j 1 constructs expressed in E.coli BL21 (DE3) (Novagen) for 24h at 37°C using auto-induction media.
Fig. 5 shows the difference between pET26-b vector pelB leader sequence and Erwinia chrysanthemi pelC leader sequence.
Fig. 6 shows the sequence of Amb a 1.3 R2. Sequence areas depicted in italic are lacking, resulting the truncated Amb a 1.3 R2.
Fig. 7 shows Amb a 1.3 R2 truncated model in black. Crystal structure of natural Jun a 1 (PDB 1pxz) was used as template structure.
Fig. 8 shows whole cell protein extract of Amb a 1.3 R2 truncate constructs expressed in E.coli BL21 (DE3) star in LBsupp media at 16°C for 20h.
Fig. 9 shows dot blot analyses of Amb a 1 and derivatives thereof expressed in E.coli. E.coli transfected with the empty expression plasmid as well as purified natural Amb a 1 were used as reference.
Fig. 10 shows ELISA analyses of of Amb a 1 and derivatives thereof expressed in E.coli. E.coli transfected with the empty expression plasmid as well as purified natural Amb a 1 were used as reference.
Fig. 11 shows mediator release analyses of Amb a 1 and derivatives thereof expressed in E.coli. E.coli transfected with the empty expression plasmid as well as purified natural Amb a 1 were used as reference.
Fig. 12 shows the comparison of codon usages between E.coli K-12 and E.coli B.

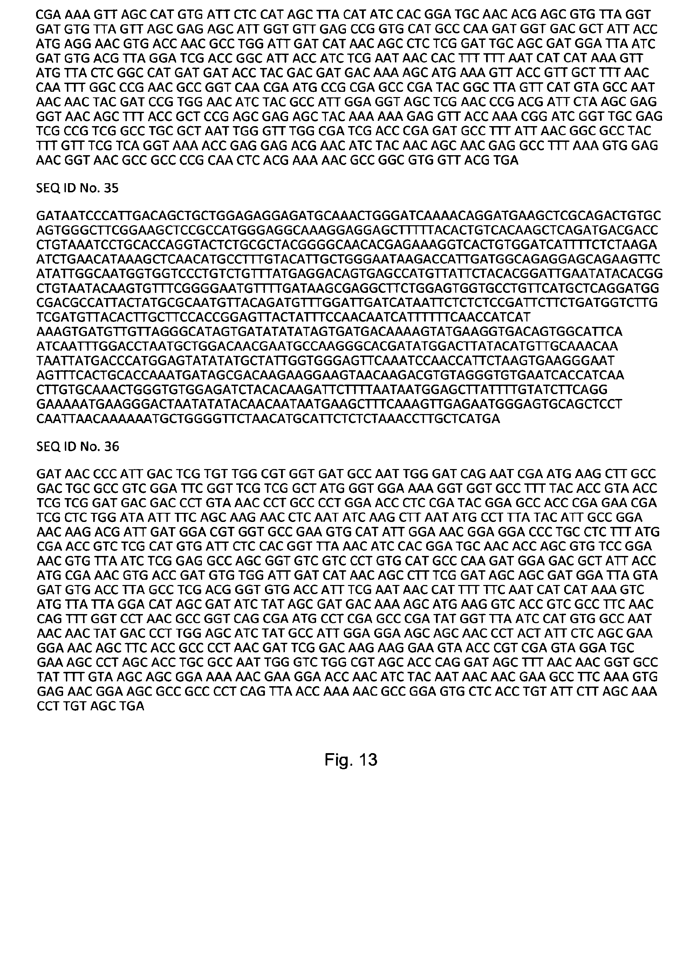

Fig. 13 shows the sequences associated to their respective sequence identifiers (SEQ ID Nos.).

### EXAMPLES:

### Material and Methods

### Patients and sera.

Ambrosia artemisiifolia, Cryptomeria japonica, Juniperus ashei, or Cupressus arizonica pollen-allergic patients were selected based on case history, positive in vivo skin prick test or in vitro IgE detection (CAP system, Phadia AB).

### Codon optimization of Amb a 1 and Cry j 1.

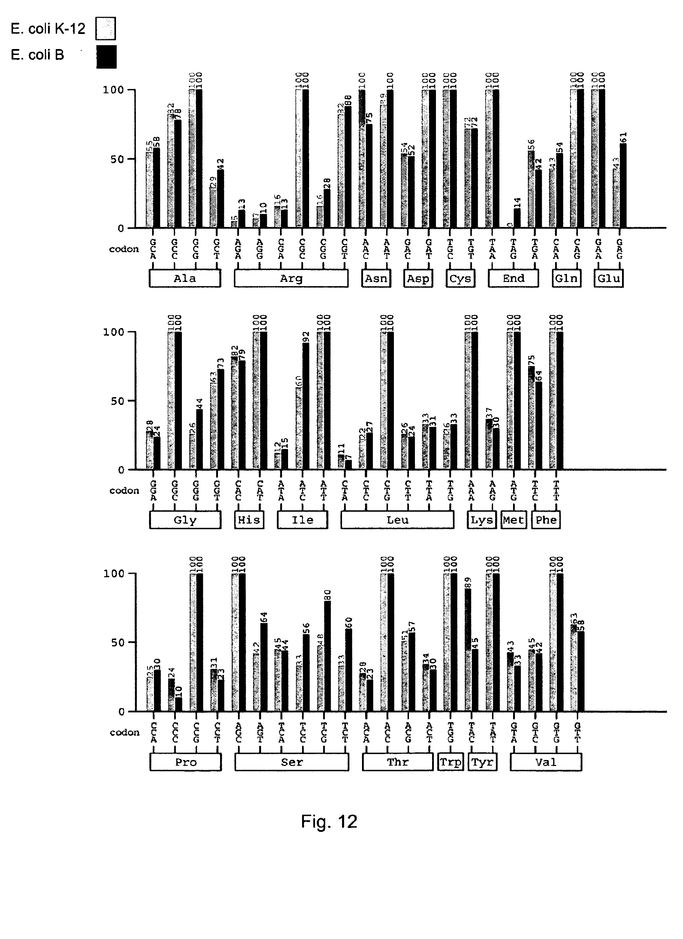

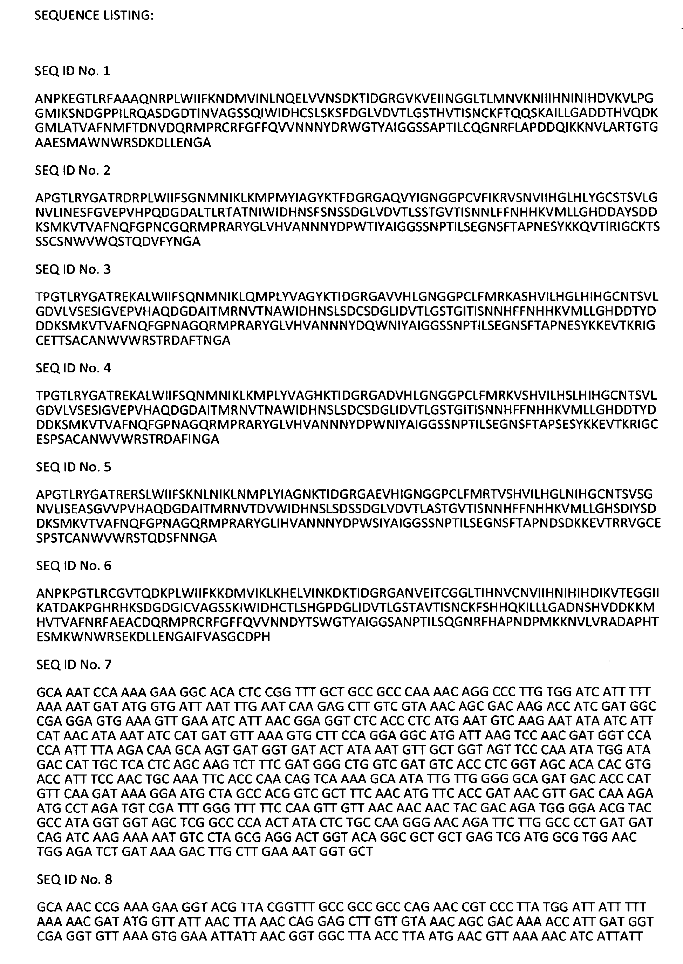

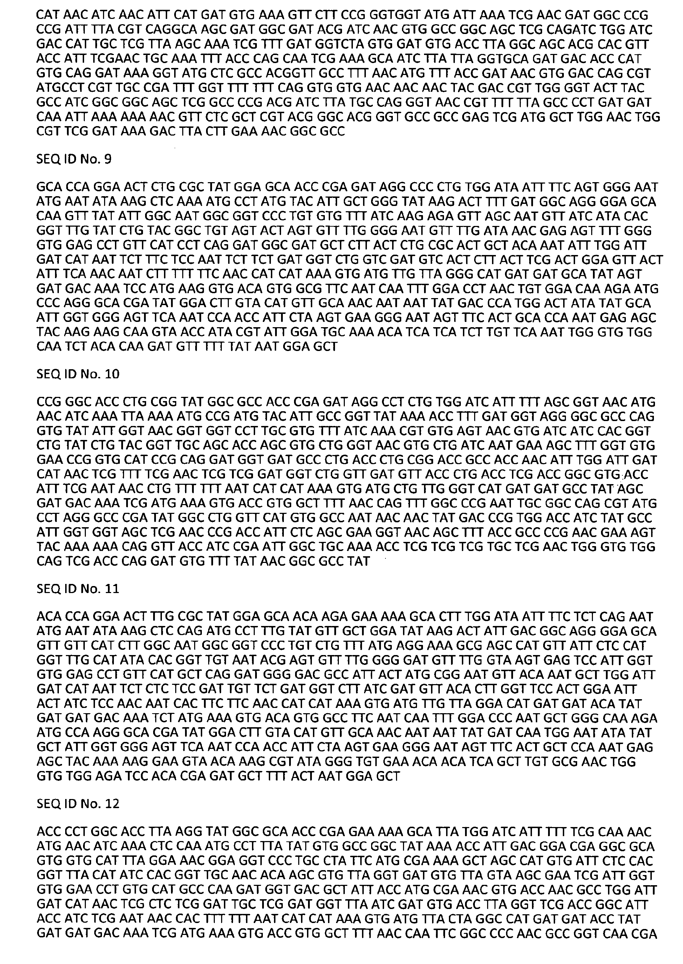

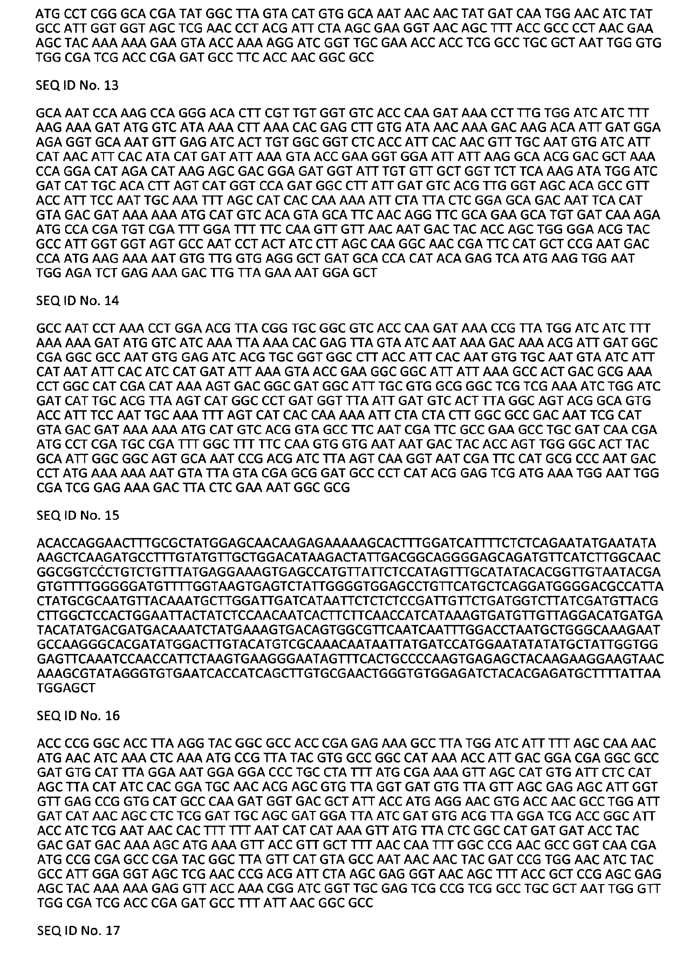

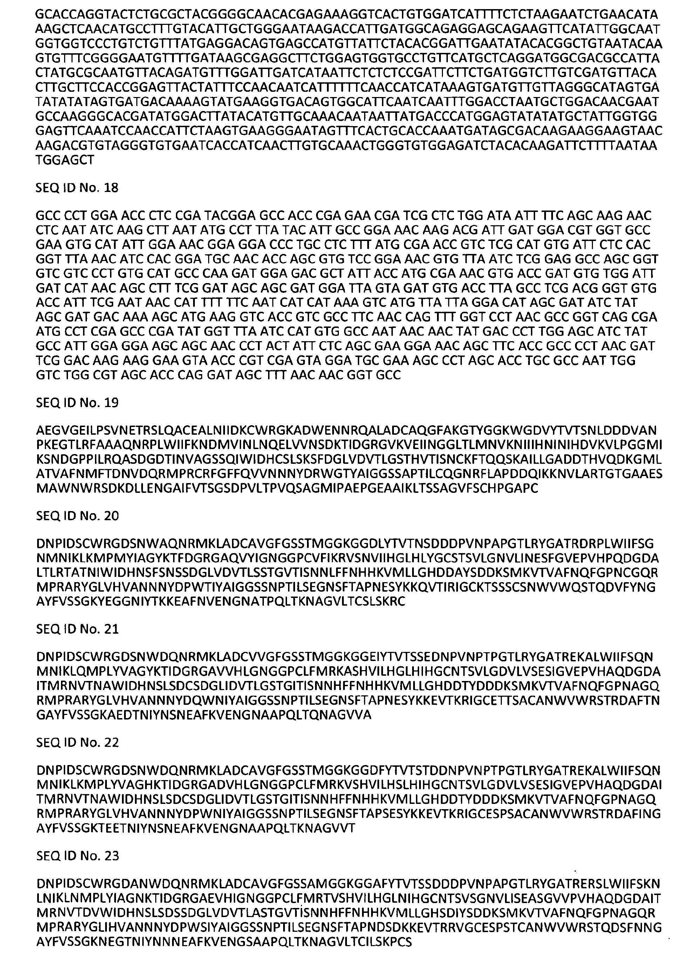

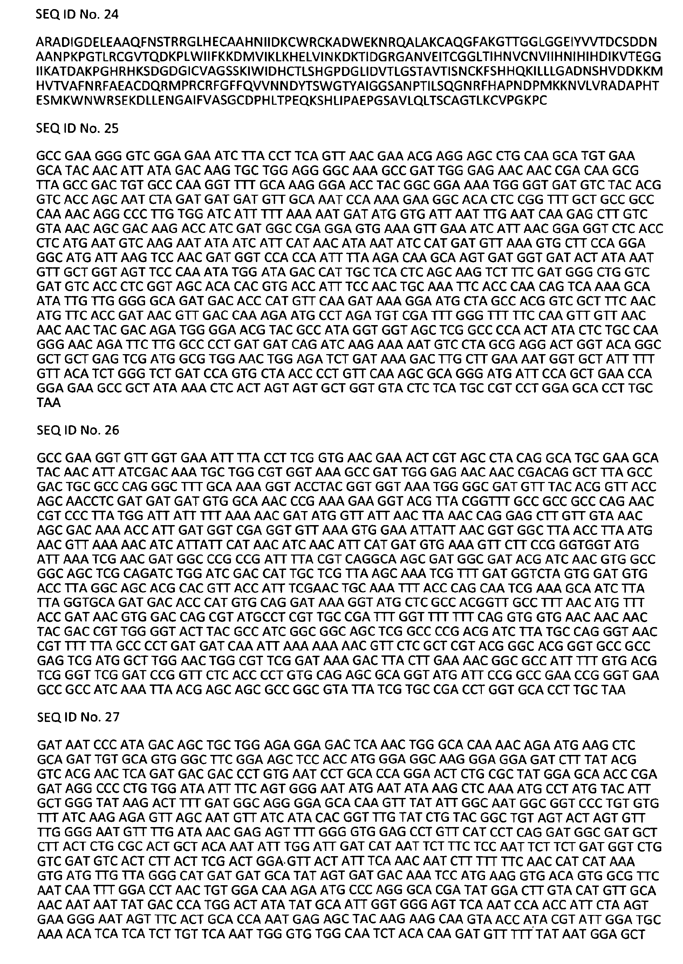

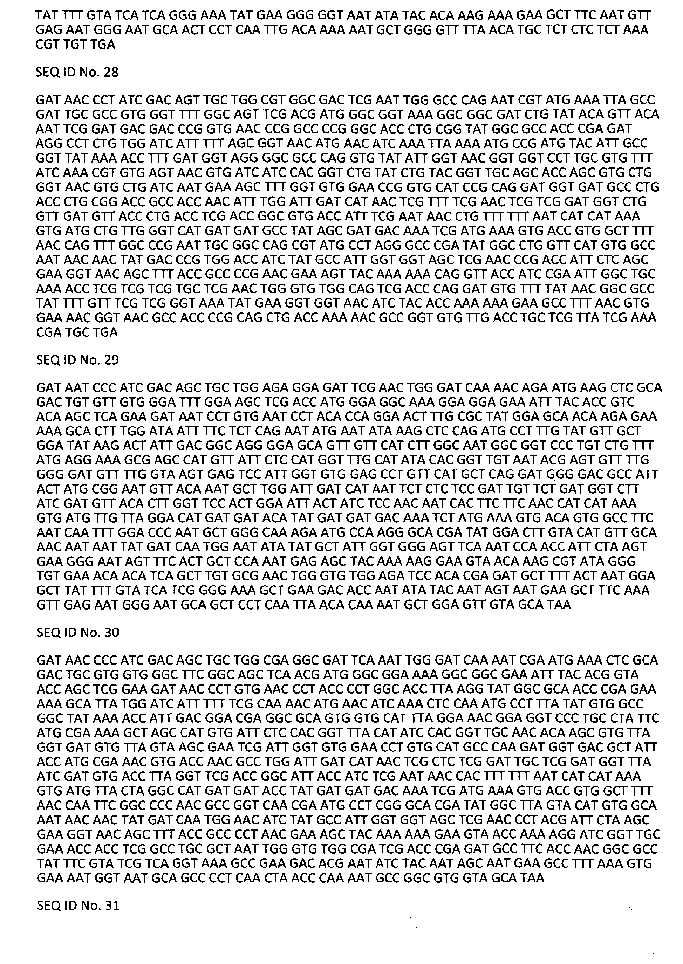

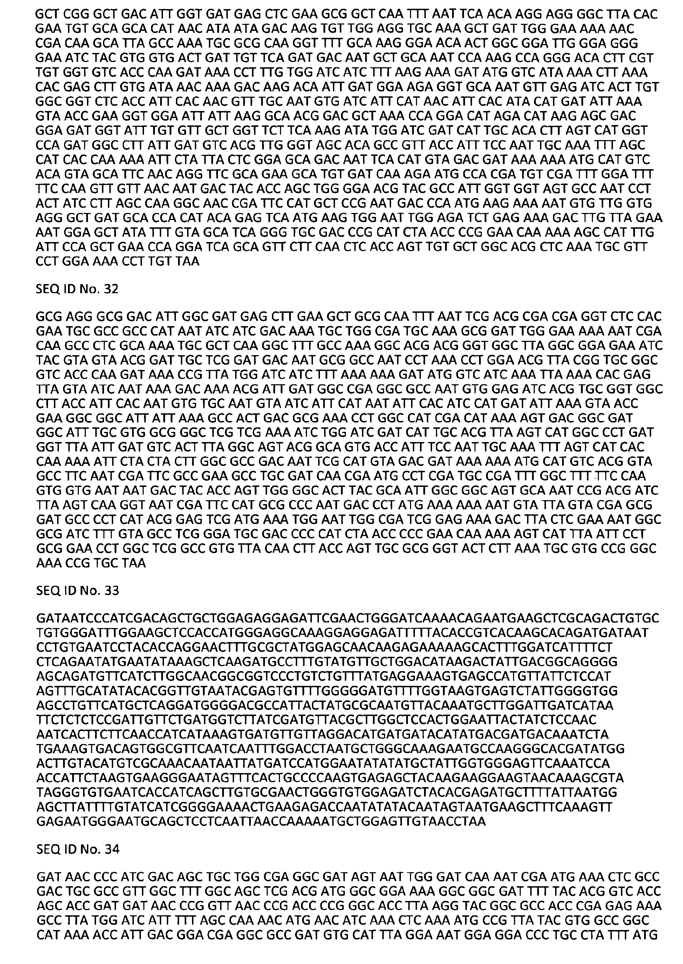

The codon usage for a given organism is available at the codon usage database http://www.kazusa.or.jp/codon/. Codon optimization of Amb a 1 and Cry j 1 were performed according to published methods (Gustafsson C. et al., Trends Biotechnol. 2004 Ju1;22 (7) : 346-53). Most of the commonly used commercially available E. coli strains are derivatives of E.coli B (e.g. BL21 and derivatives thereof, TunerTM, Origami B, Rosetta-Gami B, all available at EMD Biosciences) or E. coli K-12 (e.g. Origami 2, Rosetta 2, Rosetta-Gami 2, all available at EMD Biosciences). For protein expression the strains BL21 Star (DE3) (Invitrogen), an E. coli B derivative, and Origami 2 (EMD Biosciences), an E. coli K-12 derivative, were selected. E. coli B and K-12 are closely related (Lukjancenko O. et al., Microb Ecol. 2010 Nov; 60(4):708-20) and the codon usage of both strains is comparable. A graphical comparison of both codon usage tables was performed by a graphical codon usage analyzer (http://gcua.schoedl.de/compare_v2.html) (Figure 12). For the codon optimization the codon usage of E. coli K-12 was used and the genes of interest (e.g. Amb a 1, and Cry j 1) were codon optimized accordingly.

### Codon harmonization of Amb a 1 and Cry j 1.

The codon usage for a given organism is available at the codon usage database http://www.kazusa.or.jp/codon/. Besides codon optimization, codon harmonization has been demonstrated to be an effective tool to increase protein expression and solubility (Angov E. et al., PLoS One. 2008 May 14;3(5):e2189.; Marin M. Biotechnol J. 2008 Aug;3(8):1047-57.). In brief, rare codons slow down the translation of the mRNA allowing the newly synthetized polypeptide chain to adopt the correct conformation, thus directly affecting the folding, the structure and the biological activity of proteins. Therefore the codon usage of pectate lyase genes was determined for the respective source organism. The codon usage of the host organism was used to transfer the codon usage for the gene of interest from the source organism to the host organism. Therefore, E. coli codons encoding the respective amino acid at a given position and showing the codon usage, which was closest to the original codon of the source organism for the respective amino acid, were selected. The DNA code was then changed accordingly and the resulting gene with new codon composition was generated synthetically. For protein expression the strains BL21 Star (DE3) (Invitrogen), an E. coli B derivative, and Origami 2 (EMD Biosciences), an E. coli K-12 derivative, were selected. For the codon harmonization the codon usage of E. coli K-12 was used and the genes of interest (e.g. Amb a 1, and Cry j 1) were codon harmonized accordingly (see Fig. 12 a to e).Cloning and expression of pectate lyases and derivatives thereof.

Pectate lyase genes were PCR amplified for the original cloning vectors and subsequently cloned into pET vectors (EMD Biosciences) using the respective restriction sites. Cells were grown in LB medium to an OD600 of 0.8 at 37°C and 200 rpm on a shaker, and protein expression in was induced by the addition of 0.5 M isopropyl-β-D-thiogalactopyranosid. Expression was performed at various growth conditions (e.g. 37°C for 6 h, 16°C for 20 h) using different expression constructs as well as E. coli host strains. Thereafter, cells were harvested by low-speed centrifugation, resuspended in 25 mM TrisCl pH 9.5, 0.5 M Urea and subjected to three repeated freeze / thaw cycles at 37°C and liquid nitrogen, respectively. Thereafter, the suspension was equilibrated for 1 h at room temperature on a low-speed shaker, homogenized with an Ultra-turrax for 1 min at 14.000rpm, and centrifuged at 20.000 g for 20 min at 4°C. Soluble and insoluble protein fractions were collected for further analysis.

### ELISA experiments.

For ELISA experiments, Maxisorp plates (Nalge Nunc, Rochester, NY, USA) were coated with protein antigen (200 ng/well in 50 µl PBS) overnight at 4 °C. Plates were blocked with Tris-buffered-saline (TBS), pH 7.4, 0.05% (v/v) Tween, 1% (w/v) bovine serum albumin (BSA) and incubated with patients' sera diluted 1:10 overnight at 4 °C. Bound IgE was detected with alkaline phosphatase-conjugated monoclonal anti-human IgE antibodies (BD Biosciences), after incubation for 1 h at 37 °C and 1 h at 4 °C. 10 mM 4-nitrophenyl phosphate (Sigma-Aldrich) was used as substrate and OD was measured at 405/492 nm. All measurements were performed as duplicates; results are presented as mean OD values. Activation of basophils.

The allergenic potential of pectate lyase allergens and derivatives thereof was measured by rat basophil degranulation assay (RBL) as described (Vogel L. et al., Allergy. 2005 Aug;60(8):1021-8.). Human sera were added in a dilution of 1:15, antigen solutions were titrated and antigen-dependent beta-hexosaminidase release into the supernatant was measured by enzymatic cleavage of the fluorogenic substrate 4-methyl umbelliferyl-N-acetyl-β-glucosaminide and expressed as % of total enzyme content of Triton X100-treated cells.

### Results

### Second Amino Acid Position

The half-life of a cytosolic protein is determined to a large extent by its amino-terminal residue. A highly destabilizing residue (e.g. arginine, asparagine, aspartic acid, glutamine, glutamic acid, and leucine) favors rapid ubiquitinylation and therefore, destruction of the expressed protein, whereas stabilizing residues do not.

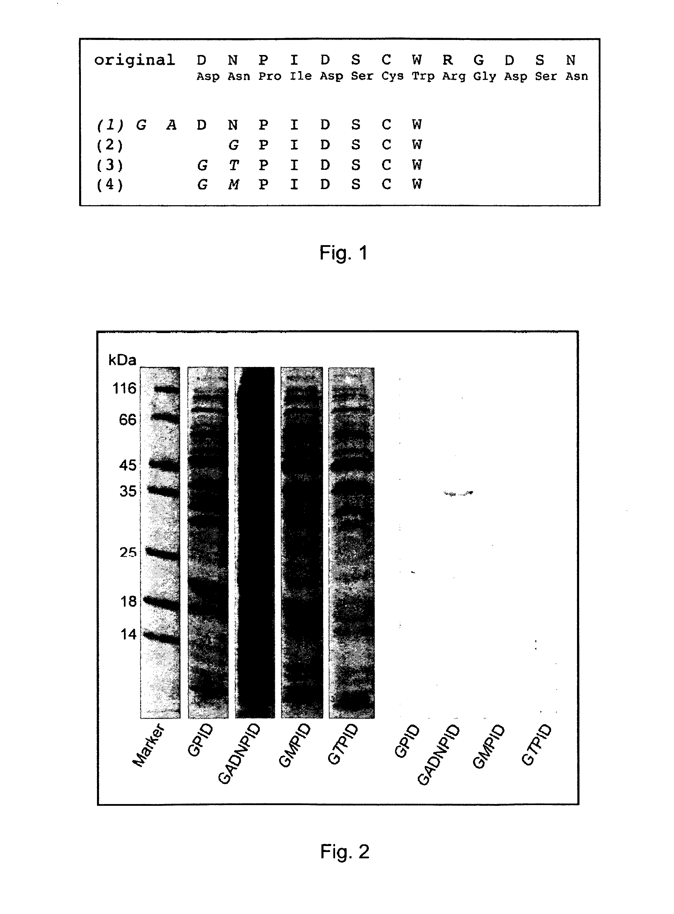

Many plant pectate lyases show a tendency to express such destabilizing amino acid residues at the mature N-terminus. To avoid destruction and to increase the expression level, 4 constructs with altered N-terminal amino acid sequence were designed (see Fig. 1).

The 4 constructs were used for protein expression in E.coli grown after induction with IPTG at OD600nm 0,7 for 7 hours at 37°C in LB supplement media. Cell breakage was performed using liquid nitrogen and resulting soluble protein fractions were analyzed by SDS-PAGE and Western Blot (see Fig. 2).

The Cry j 1 construct with additional Gly-Ala at the N-terminus shows higher total protein expression in E.coli, when compared with the other constructs. Furthermore, when using the Cry j 1 construct possessing Alanine at the second amino acid position, expression of recombinant Cry j 1 was clearly detectable via Western Blot.

This observation is in accordance with E.coli protein expression studies, showing that high expressed proteins have an increased frequency of alanine in the second amino acid position.

### Codon Harmonization

The process of codon optimization for protein production is a widely used technique in molecular biology. However, during heterologous protein expression, low expression levels or formation of insoluble aggregates may be attributable to differences in synonymous codon usage between expression and natural host. In contrast, codon harmonization uses synonymous codons from E.coli that match as closely as possible the codon usage frequency used in the native gene. Several studies could demonstrate that the approach of codon harmonization significantly improved protein yield and protein solubility (Angov E, et al. PloSOne 3 (2008) :e2189).

Therefore, a codon optimized and a codon harmonized version of the Cry j 1-gene were designed and compared in protein expression levels.

Escherichia coli K-12 codon usage data were used, as Origami and BL21 (DE3) strains used for protein expression are derivatives of strain K-12.

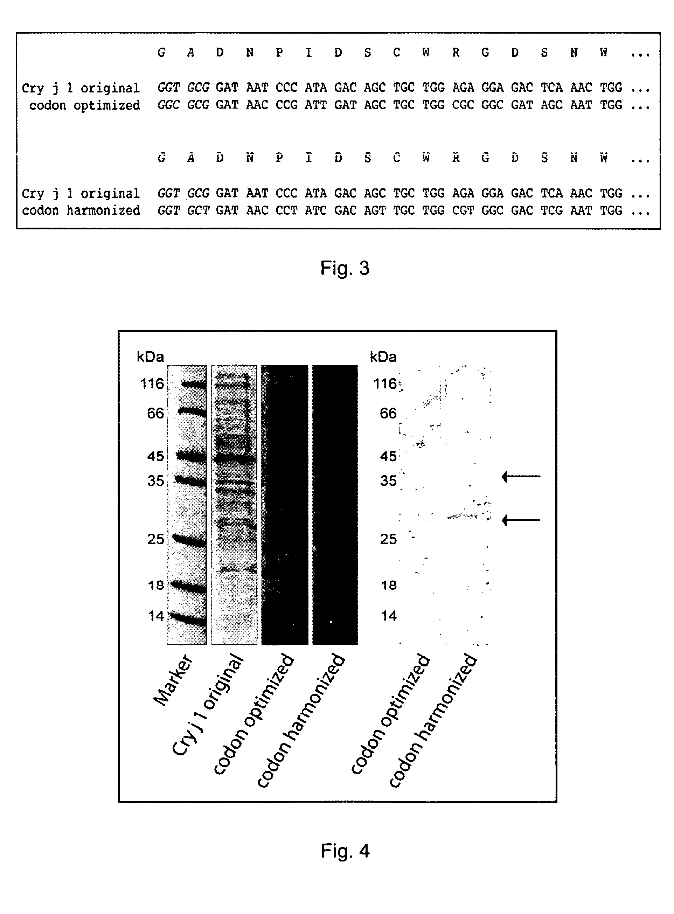

For protein expression, original Cry j 1 as well as Cry j 1 codon optimized and Cry j 1 harmonized were compared (see Fig. 4).

Expression of original, codon optimized, and codon harmonized Cry j 1 was performed using LB media as well as auto-induction media. Improved expression results could be monitored when using auto-induction media and the Cry j 1 codon harmonized construct (see Fig. 4). However, very little amount of the protein expressed seems to be full length Cry j 1; most of the protein is degraded (around 27kDa in the immunoblot). This seems to be a possible "alpha chain", as known for the ragweed major allergen Amb a 1 (Wopfner N, et al. Molecular Immunology 46(2009):2090-2097).

### Leader Sequence

Optimization of the second amino acid position and codon harmonization were shown to afford soluble expression of recombinant Cry j 1 constructs. To increase the protein expression level, an expression vector including a leader sequence for potential periplasmic localization of the recombinbantly produced protein. The vector used was pET26b (Novagen) combining the formerly used pET 28b vector and a pelB leader sequence (pelB from pectate lyase B).

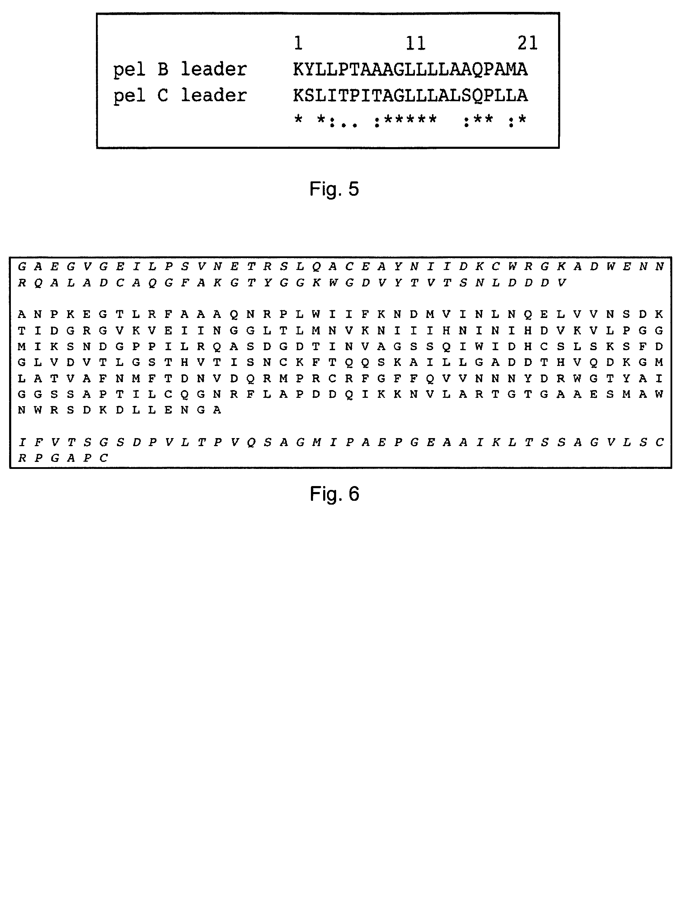

However, additional investigations regarding the family tree of all known pectate lyases revealed allergenic plant pectate lyases like Cry j 1, Amb a 1, and Cup a 1 to be more related with pectate lyases C than pectate lyases B (pectate lyases included are from CAZY - Carbohydrate-active enzymes database, for pectate/pectin lyases n=178; see Fig. 5). Therefore, the pelC leader sequence from Erwinia crysanthemi was used instead of the pelB leader sequence. Furthermore, to investigate if this approach is also applicable for other pectate lyases, Amb a 1 was used as template.

### Truncation

One objective was to generate a pectate layse hypoallergen for use in immunotherapy. However, the so far described approaches afford soluble expression of full length pectate lyases, i.e. Amb a 1, Cry j 1, and Cup a 1.

The family of pectate lyases, independent from which organism, feature one crucial structural similarity, the beta helix. A folding motif composed of three parallel beta strands forming a large right-handed helix, shown to be a very stable structural motif (Yoder MD, et al. The FASEB Journal 9(1995):335-342). The various known pectate lyases differ in size and conformation of loops that protrude from the central beta helix core. Therefore, a truncated version of Amb a 1, Cry j 1, and Cup a 1 was designed: lacking N- and C-terminal parts of the wild type protein, leaving the entire beta helix with the known T-cell epitopes.

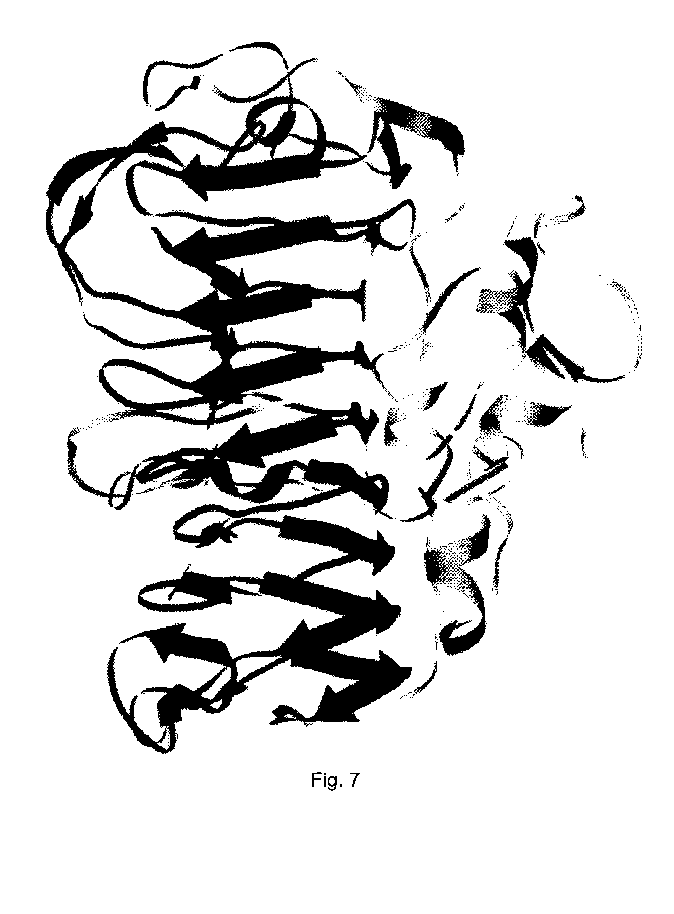

Figs. 6 and 7 show the differences in sequence and structure between Amb a 1.3 alpha chain and Amb a 1.3 truncate. Protein expression of the different constructs was performed using E.coli BL21 (DE3) star in LBsupp media and incubation at 16°C for 20 hours.

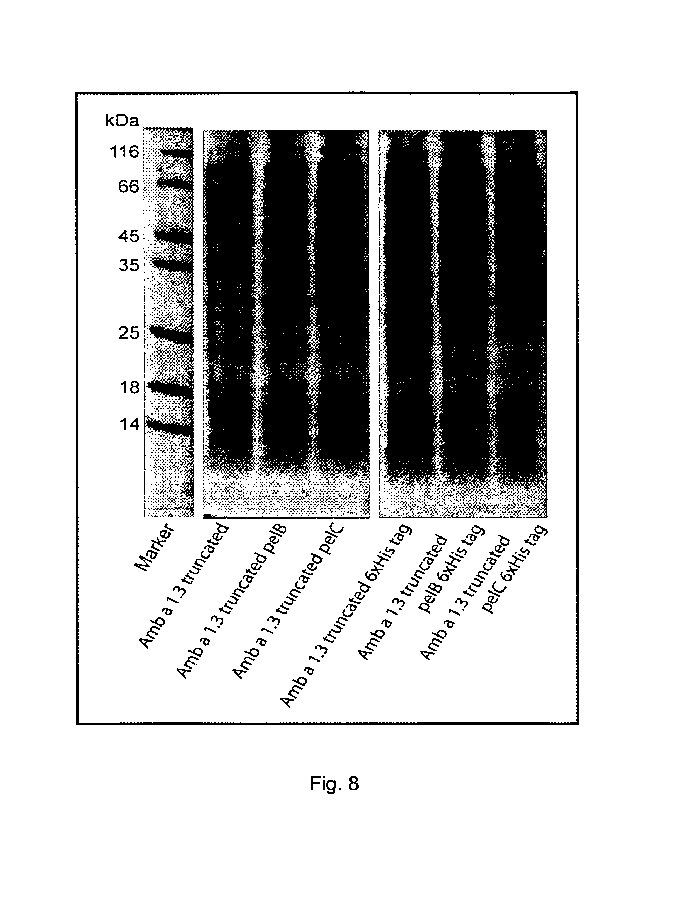

As depicted in Fig. 8, the Amb a 1.3 truncate construct with the Erwinia crysanthemi pelC leader sequence shows highest level of protein expression. Therefore, all following constructs (Cry j 1, Cup a 1, either full length or truncate versions) were cloned using the pelC leader.

### Patients IgE binding

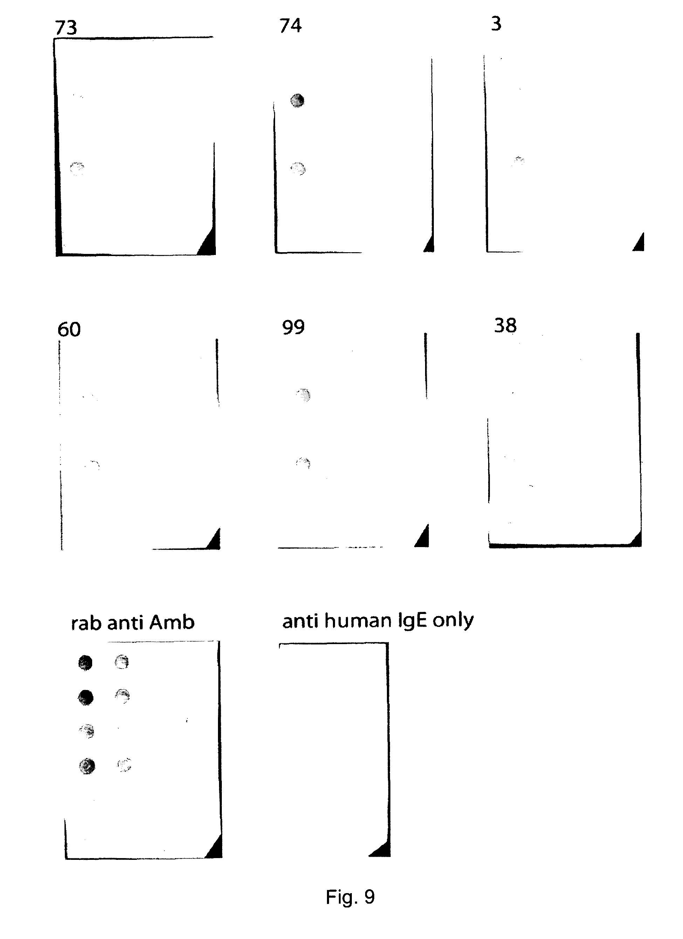

### DotBlot (see Fig. 9) :

Patients Sera CE 2009, Patients 73, 74, 3, 60, 99, 38
● Proteins: Amb Trunc Pel C fresh expressed, soluble lysate, Trunc content verified by SDS-Page, 2.9 mg/ml total protein, Trunc estimated 10% of total protein
● Amb FL Pel C freshly expressed, soluble lysate, FL content verified by SDS-Page, 2.8 mg/ml total protein, FL estimated 10% of total protein
● Amb alpha chain fresh expressed, 6M Urea, alpha chain content verified by SDS-Page, 5.7 mg/ml total protein, alpha chain estimated 10% of total protein
● Amb R2 fresh expressed, 6M Urea, R2 content verified by SDS-Page, 3.2 mg/ml total protein, R2 estimated 10% of total protein
● BL21 induced with IPTG, no plasmid, soluble fraction
● 3.6 mg/ml total protein nat Amb a 1 - conc. 0.2 mg/ml determined by Bradford assay

Spotted 50µl per spot, sera were diluted 1: 10, oN incubation at 4°, detection with anti human IgE AP BD Pharmingen 1: 5.000, 4h RT, substrate NBT / BCIP

Rabbit anti nat Amb a 1 dilution 1: 10.000, oN incubation at 4°, detection with goat anti rabbit AP Jackson 1: 10.000, 4h RT, substrate NBT / BCIP

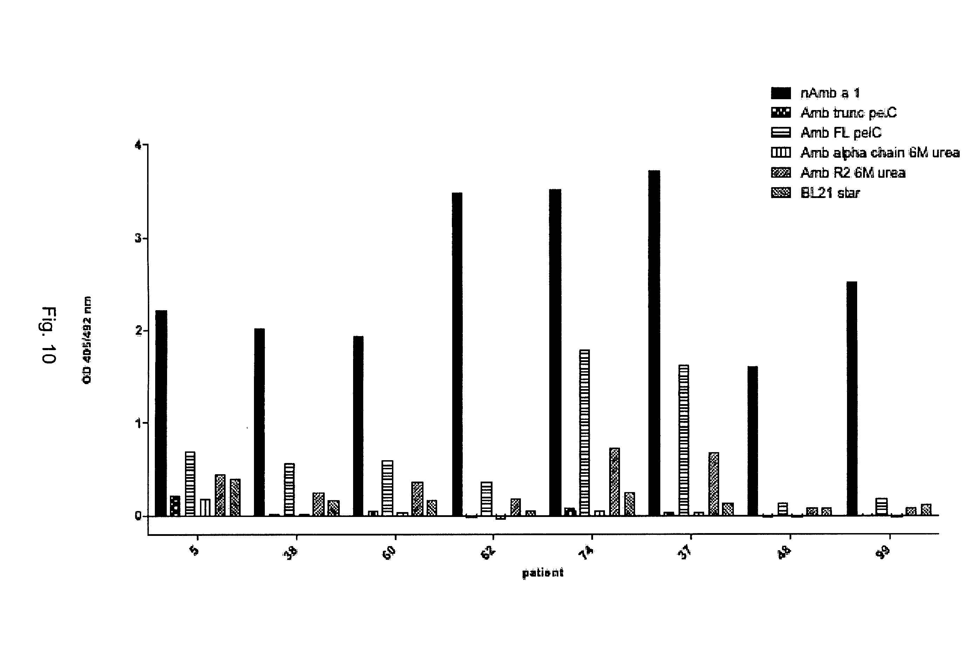

### ELISA (see Fig. 10)

Patients sera used: CE sera 5,38,60,62,74,37,48,99

Coating conditions: 25µg/ml total protein from bacterial lysates 2µg/ml purified natural Amb a 1 (nAmb a 1)

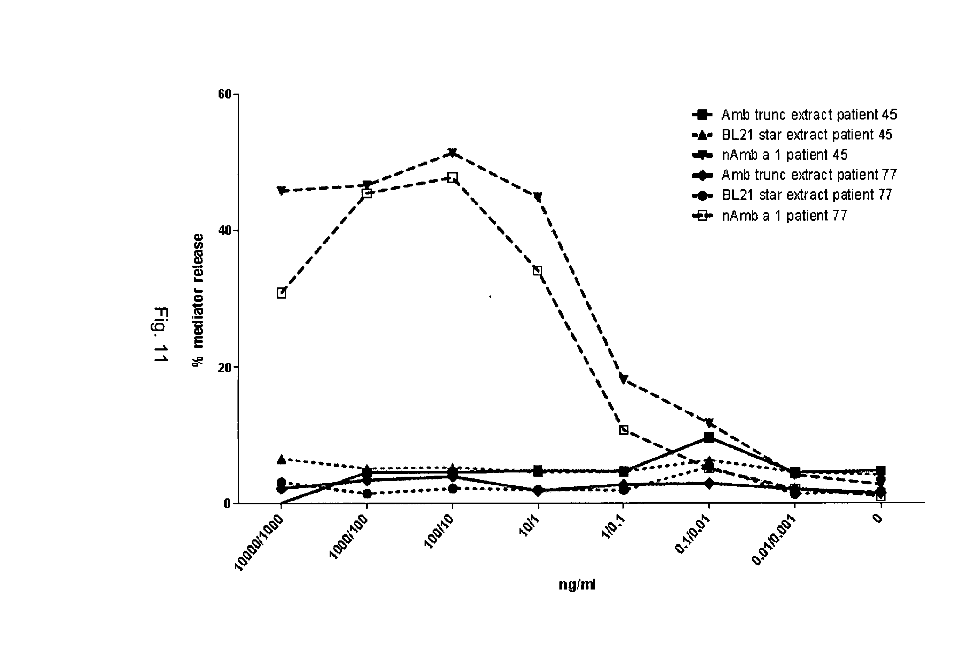

RBL - mediator release assay (See Fig. 11)

Patients Sera CE 2009, Patients 45, 77

### Proteins:

● nat Amb a 1 - 0.2 mg/ml
● Amb Trunc fresh expressed, soluble lysate, Trunc content verified by SDS-Page, 2.5 mg/ml total protein, Trunc estimated 10% of total protein BL21 induced with IPTG, no plasmid, soluble fraction, 3.5 mg/ml total protein

The protein concentration of Amb a 1 was 10 times less, since in the extracts only approx. 10% Amb Trunc were present.

Only purified natural Amb a 1 could show mediator release. Amb a 1 trunc did not show mediator release reflecting the significant reduction in allergenicity. As control, BL21, no plasmid, soluble fraction was used.

## Claims

1. A variant of a mature polysaccharide lyase having a beta helix structure, wherein said variant comprises an N- and/or C-terminal truncation and consists essentially of at least a part of the beta helix structure of the mature polysaccharide lyase.

2. Variant according to claim 1, **characterised in that** the N-and/or C-terminal truncation comprises at least 10 amino acid residues.

3. Variant according to claim 1 or 2, **characterised in that** said N-terminal truncation comprises the region extending from the N-terminal end of the polysaccharide lyase to approximately 1 to 50 amino acid residues before the N-terminus of the first beta sheet being part of the beta helix structure and said C-terminal truncation comprises the region extending from the C-terminal end of the polysaccharide lyase to approximately 1 to 50 amino acid residues after the C-terminus of the last beta sheet part of the beta helix structure, under the provisio that the first and last beta sheet of the beta helix structure remains intact.

4. Variant according to any one of claims 1 to 3, **characterised in that** the polysaccharide lyase is a pectate lyase, preferably an allergenic pectate lyase.

5. Variant according to claim 4, **characterised in that** the allergen is selected from the group consisting of Amb a 1, , Art v 6, Cha o 1, Cup a 1, Cry j 1 and Jun a 1.

6. Variant according to claim 5, **characterised in that** the variant derived from Amb a 1 consists essentially of amino acid sequence SEQ ID No: 1, the variant derived from Cry j 1 consists essentially of amino acid sequence SEQ ID No: 2, the variant derived from Cup a 1 consists essentially of amino acid sequence SEQ ID No: 3, the variant derived from Jun a 1 consists essentially of amino acid sequence SEQ ID No: 4, the variant derived from Cha o 1 consists essentially of amino acid sequence SEQ ID No: 5 and the variant derived from Art v 6 consists essentially of amino acid sequence SEQ ID No: 6.

7. Polypeptide comprising a secretion signal sequence fused directly or via a linker to a variant according to any one of claims 1 to 6 or to a mature polysaccharide lyase, preferably as defined in claim 4 or 5, or a fragment thereof.

8. Polypeptide according to claim 7, **characterised in that** the secretion signal sequence is selected from the group consisting of a PelB, a PelC, a OmpA, a StII, an EX, a PhoA, a OmpF, a PhoE, a MalE, an OmpC, a LPP, a LamB, an OmpT, a LTB, a phosphatase (pho1) and an invertase (Suc) secretion signal sequence and an α-mating factor prepro-sequence.

9. Polypeptide according to claim 7 or 8, **characterised in that** the linker comprises a protease cleavage site.

10. Polypeptide according to claim 9, **characterised in that** the protease cleavage site is capable of being cleaved by thrombin, chymotrypsin, trypsin, pepsin, subtilisin, enterokinase, factor Xa, TEV protease and PreScission protease, whereby TEV protease is particularly preferred.

11. Nucleic acid molecule encoding a variant of a polysaccharide lyase according to any one of claims 1 to 6 or a polypeptide according to any one of claims 7 to 10.

12. Nucleic acid molecule according to claim 11, **characterised in that** the nucleotide sequence of said nucleic acid molecule is codon optimised and/or codon harmonised.

13. Nucleic acid molecule according to claim 11 or 12, **characterised in that** the nucleic acid molecule encoding the variant of Amb a 1 has the nucleotide sequence SEQ ID No: 7 or SEQ ID No: 8, the nucleic acid molecule encoding the variant of Cry j 1 has the nucleotide sequence SEQ ID No: 9 or 10, the nucleic acid molecule encoding the variant of Cup a 1 has the nucleotide sequence SEQ ID No: 11 or 12, the nucleic acid molecule encoding the variant of Art v 6 has the nucleotide sequence SEQ ID No: 13 or 14, the nucleic acid molecule encoding the variant of Jun a 1 has the nucleotide sequence SEQ ID No: 15 or 16 and the nucleic acid molecule encoding Cha o 1 has the nucleotide sequence SEQ ID No: 17 or 18.

14. Vector comprising a nucleic acid molecule according to any one of claims 11 to 13.

15. Method for the recombinant production of a polysaccharide lyase variant having a beta helix structure according to any one of claims 1 to 6 or a polypeptide according to any one of claims 7 to 10 comprising the steps of:
a) cultivating a host cell comprising a nucleic acid molecule according to any one of claims 11 to 13 or a vector according to claim 14 in a culture medium,
b) isolating the polysaccharide lyase variant or polypeptide from the supernatant of the culture medium.

16. Method according to claim 15, **characterised in that** the secretion signal sequence is removed from the polysaccharide lyase variant or polypeptide by incubating the lyase variant or polypeptide of step b) to a protease capable to cleave the secretion signal sequence from the lyase variant of a polypeptide.
